(19) Europäisches Patentamt
European Patent Office
Office européen des brevets

(11) EP 1 648 529 B1

(12) EUROPÄISCHE PATENTSCHRIFT

(45) Veröffentlichungstag und Bekanntmachung des Hinweises auf die Patenterteilung:
**30.05.2007 Patentblatt 2007/22**

(21) Anmeldenummer: **04732292.0**

(22) Anmeldetag: **12.05.2004**

(51) Int Cl.:
***A61L 15/26*** (2006.01)

(86) Internationale Anmeldenummer:
**PCT/EP2004/005052**

(87) Internationale Veröffentlichungsnummer:
**WO 2005/004936 (20.01.2005 Gazette 2005/03)**

(54) **HAUT- ODER WUNDAUFLAGE MIT VERKAPSELTEN, WUNDHEILUNGSFÖRDERNDEN UND/ ODER HAUTPFLEGENDEN SUBSTANZEN**

SKIN OR WOUND PAD CONTAINING ENCAPSULATED SUBSTANCES WHICH PROMOTE THE HEALING OF WOUNDS AND/OR ARE USED FOR SKIN CARE

PANSEMENT APPLICABLE SUR LA PEAU OU SUR DES PLAIES, RENFERMANT DES SUBSTANCES ENCAPSULEES FAVORISANT LA GUERISON DES PLAIES ET/OU POUR SOINS CUTANES

(84) Benannte Vertragsstaaten:
**AT BE BG CH CY CZ DE DK EE ES FI FR GB GR HU IE IT LI LU MC NL PL PT RO SE SI SK TR**

(30) Priorität: **08.07.2003 DE 10330971**

(43) Veröffentlichungstag der Anmeldung:
**26.04.2006 Patentblatt 2006/17**

(73) Patentinhaber: **Beiersdorf AG**
**20253 Hamburg (DE)**

(72) Erfinder:
- **JÄNICHEN, Jan**
**22149 Hamburg (DE)**
- **SACHAU, Günther**
**25451 Quickborn (DE)**
- **HARTKOPF, Carsten**
**22303 Hamburg (DE)**
- **BERG, Thorsten**
**20149 Hamburg (DE)**

(56) Entgegenhaltungen:
**EP-A- 1 190 722** **EP-A- 1 190 723**
**WO-A-02/100450** **WO-A-02/102358**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft eine Haut- oder Wundauflage mit wundheilungsfördernden und/oder hautpflegenden Substanzen, die in verkapselter Form in der Auflage vorliegen und erst beim Kontakt mit dem Wundsekret oder Feuchtigkeit die Substanz kontrolliert abgeben.

Die richtige galenische Formulierung von physiologisch aktiven Substanzen gehört zu den Hauptaufgaben des Pharmazeuten in der Arzneimittelindustrie. So macht der eigentliche Wirkstoff in der pharmazeutischen Formulierung oft nur einen kleinen Teil der Gesamtrezeptur neben zahlreichen physiologisch unwirksamen Hilfsstoffen aus. Jedoch sind es gerade diese Hilfsstoffe, über die Freisetzungsort und -kinetik einer aktiven Substanz im Organismus so modifiziert werden können, dass eine optimale Entfaltung der gewünschten Wirkung realisiert wird. So kann durch säureresistente Kapseln erreicht werden, dass Wirkstoffe den sauren Magen passieren, .bevor sie gezielt im alkalischen Darmmilieu freigesetzt werden, Retardformulierungen nutzen die diffussionskontrollierte Freisetzung des Wirkstoffs aus dem Pharmazeutikum aus, um den Wirkstoff über einen längeren Zeitraum in pharmakologisch aktiven Konzentrationen anzubieten (siehe hierzu auch: K.H. Bauer, K-H. Frömming, C. Führer in "Pharmazeutische Technologie", 5. Auflage 1997).

Hautverletzungen und Wunden stellen für die Entwicklung geeigneter Formulierungen wundheilungsfördernder Substanzen eine interessante Herausforderung dar, da neben einer hohen Stoffwechseltätigkeit in der Wunde eine stetige Sekretion von Wundflüssigkeit für ein sich kontinuierlich änderndes Wundmilieu sorgt. Formulierungen mit wundheilungsfördernden und desinfizierenden Substanzen sind vor allem auf Basis von flüssigen Rezepturen oder Salben bekannt und müssen zur optimalen Wrkungsentfaltung mehrfach täglich aufgebracht werden.

Die Versorgung von Wunden mit Wundpflastern und -verbänden hat primär das Ziel ein mechanisches Eindringen von Fremdkörpern und Keimen zu verhindern und ein Wundmilieu zu schaffen, in dem optimale Bedingungen für den Heilungsprozess der Haut herrschen.

Moderne Wundversorgungsprodukte wie Hydrokolloide (siehe hierzu zum Beispiel "Hydrokolloide" von R. Lipmann in "Medical Device & Diagnostic Industry, June 1999), die für Kolostomie- und professionelle Wundversorgungsanwendungen entwickelt wurden, finden zunehmend Anwendung.

Wundversorgungsprodukte auf Basis von Hydrokolloiden weisen Vorteile gegenüber herkömmlichen Pflastern auf. Diese generieren ein feuchtes Wundheilungsmilieu, das die Wunde nicht austrocknen lässt und ein optimales Milieu zur schnellen Wundheilung erzeugt. Weitere Vorteile sind die Unauffälligkeit bei der Anwendung, sicheres Haftvermögen, Absorptionsvermögen von Exudat, gute Polsterwirkung und die schmerzlose Entfernbarkeit.

Konturierte Wundauflagen mit einer Klebmasseschicht bestehend aus quellbaren Hydrokolloiden und wasserunlöslichen, viskosen Bestandteilen, beispielsweise Polyisobutylen, Kautschuk, Silicon oder Polyurethanelastomeren, sind Gegenstand der WO 92/05755.

Wasserfreie Hydrogele werden als Xerogele bezeichnet und sind makromolekulare, natürliche oder synthetische Stoffe, die aufgrund eines hohen Gehaltes an hydrophilen Gruppen in der Lage sind, Wasser absorptiv zu binden. Die Wasseraufnahmekapazität vieler Xerogele beträgt das Mehrfache des Eigengewichtes der wasserfreien Substanz. Hydrogele oder Xerogele werden in vielfältiger Form in der Wundversorgung eingesetzt, denn sie schützen Wunden vor der Austrocknung, saugen Wundsekret auf, dienen als Matrix für Wirkstoffe aller Art und auch als Basis für die Besiedelung mit autologen oder heterologen Hautzellen.

Gele können unter anderem in Form von Schäumen verwendet werden. Schäume zur Versorgung von Hautwunden oder chirurgischen Wunden sind dem Fachmann an sich bekannt. Hauptsächlich finden dabei Polyurethanschäume oder Kollagenschäume Verwendung.

Auch selbstklebende Gelschäume sind dem Fachmann bekannt. Diese lassen sich zwar im allgemeinen recht gut auf der Haut fixieren, haben aber meistens den Nachteil, dass ihre Wasseraufnahmekapazität und ihr Wasserrückhaltevermögen stark eingeschränkt sind.

Weiterhin sind hydrophile Schäume aus Polyurethangelen bekannt. Die WO 88/01878 A1 beschreibt selbstklebende Polyurethanschäume beziehungsweise Polyurethanschaumgele, welche unter anderem einpolymerisierte Methacrylate enthalten können. Die Herstellung dieser Schaumgele erfolgt durch Zusatz von Wasser.

Polyurethangele auf der Basis einer Polyurethanmatrix und höhermolekularen Polyolen werden auch in EP 0 057 839 B1 beschrieben. Selbsthaftende Flächengebilde aus Polyurethangelen sind aus EP 0 147 588 B1 bekannt. Die in diesen beiden letztgenannten Schriften offenbarten Polyurethangele sind ungeschäumt. Die selbstklebenden Gele haben Isocyanatkennzahlen von 15 bis 70 (EP 0 147 588 A2).

EP 0 196 364 A2 beschreibt hydrophile Polyurethanschäume, die mit wasserabsorbierenden Polymeren auf Basis eines Copolymers der Acrylsäure und Kaliumacetat gefüllt sein können und für medizinische Zwecke gedacht sind. Das Polyurethan wird auf Basis von MDI, Methylendiphenyldiisocyanat, hergestellt. Der eingesetzte Polyether hat eine Mindestfunktionalität von zwei Hydroxylgruppen, bevorzugt je zwei bis drei Hydroxylgruppen. Das Verhältnis NCO/OH ist stöchiometrisch. Damit handelt es sich nicht um ein gelförmiges Polyurethan. Geschäumt werden kann mit Druckluft oder mit anderen, nicht mit dem Isocyanat reagierenden Gasen oder mit Hilfe von niedrig siedenden Lösungsmitteln. Die Mischung von wasserabsorbierenden Polymeren mit Polyetherpolyol erfolgt etwa im Verhältnis 3 : 1 und

dient zur Wasseraufnahme. Der Schaum hat klebende Eigenschaften auf Wunden, die durch ein aluminisiertes Vlies gänzlich unterdrückt werden müssen, um ihn zur Wundbehandlung einsetzen zu können. Die in EP 0 196 364 A2 offenbarten wasserabsorbierenden Polymere sind nicht mit Wirkstoffen dotiert.

Schaumwundauflagen, bestehend aus einem Polyurethangelschaum aus einem Polyadditionsprodukt eines Polyetherpolyols (Levagel ® von Bayer AG) mit einem aromatischen oder aliphatischen Diisocyanat (Desmodur ® Bayer AG), in das ein Polyacrylat-Superabsorber-Pulver (Favor®, Stockhausen) eingearbeitet wurde, sind u. a. beschrieben in DE 42 33 289 A1, in DE 196 18 825 A1 und WO 97/43328. Das Polyurethangel kann, je nach Verhältnis von OH-Äquivalenten des Polyols zu reaktiven Isocyanat-Gruppen, schwach oder stark selbsthaftend auf Haut eingestellt werden. Es ist bekannt, dass die Einarbeitung wasserabsorbierender Polymere in die reaktiven Vorstufen einer Polyurethanreaktion die Herstellung von Polyurethan basierten Wundauflagen ermöglicht, die eine ausgezeichnete Hautverträglichkeit sowie eine hohe Kapazität zur Aufnahme von Flüssigkeit haben. Das durch solche Wundauflagen geförderte feuchte Wundmilieu trägt zu einer erheblichen Beschleunigung der Wundheilung bei. In keiner der Druckschriften des Standes der. Technik werden jedoch mit wundheilungsfördernden Wirkstoffen oder mit hautpflegenden Substanzen dotierte Absorber in Polykondensat- insbesondere Polyurethanmatrizes offenbart.

Darüber hinaus sind wirkstoffhaltige Pflastersysteme als Transdermale Therapeutische Systeme (TTS) zur Abgabe von Wirkstoffen durch die Haut seit langer Zeit bekannt. Die topische Applikation von Arzneimitteln über wirkstoffhaltige Pflastersysteme bietet zwei Hauptvorteile: Erstens wird durch diese Darreichungsform eine Freisetzungskinetik des Wirkstoffes realisiert, mit der über einen längeren Zeitraum eine Wirkstoffversorgung des Organismus aufrechterhalten werden kann. Zweitens werden über den Aufnahmeweg durch die Haut der Magen-Darm-Trakt sowie die erste Leberpassage vermieden. Dadurch können ausgewählte Arzneistoffe in einer geringen Dosierung wirkungsvoll verabreicht werden. Dies ist insbesondere dann von Vorteil, wenn eine lokale Wirkung des Arzneistoffes unter Umgehung einer systemischen Wirkung erwünscht ist. Dies ist zum Beispiel bei der Behandlung rheumatischer Gelenkbeschwerden oder Muskelentzündungen der Fall.

Eine in der Fachliteratur gut beschriebene Ausführungsform solcher transdermalen Systeme stellen Matrixsysteme oder monolitische Systeme dar, in denen der Arzneistoff direkt in den druckempfindlichen Haftklebstoff eingearbeitet wird. Eine solche haftklebrige, wirkstoffhaltige Matrix ist im anwendungsfertigen Produkt auf der einen Seite mit einem für den Wirkstoff undurchlässigen Träger ausgestattet, auf der gegenüberliegenden Seite befindet sich eine mit einer Trennschicht ausgestattete Trägerfolie, die vor der Applikation auf die Haut entfernt wird (kleben&dichten, Nr.42, 1998, S. 26 bis 30). So wird vornehmlich die Verwendung von Polyacrylaten und/oder Polyurethanen als Basis der haftklebrigen Polymermatrix erwähnt (Lamba, Woodhouse, Cooper, "Polyurethanes in Biomedical Applications", CRC Press, 1998, S. 240) und WO 01/68060.

Ein Problem bei der Herstellung transdermaler therapeutischer Systeme ist das Einbringen polarer Wirkstoffe in die zumeist unpolaren Polymermatrizes. Dadurch können bevorzugte Wirkstoffe mitunter nur schlecht oder nur in begrenzter Konzentration in die Polymermatrix eingearbeitet werden. Des weiteren besteht die Gefahr, dass aufgrund der unterschiedlichen Polarität und Unlöslichkeit der Wirkstoffe in der Polymermatrix die Wirkstoffe mit der Zeit aus dem Polymersystem auskristallisieren. Eine Langzeitstabilität ist damit nicht immer gewährleistet.

Bei der Einarbeitung verschiedener wundheilungsfördernder oder hautpflegender Substanzen in insbesondere Polyurethanmatrizes besteht darüber .hinaus das Problem, dass viele dieser Substanzen mit polaren funktionellen Gruppen, wie beispielsweise Dexpanthenol, in der Vernetzungsreaktion des Polyurethans mitreagieren. Dadurch kommt es zum einen zu einer gestörten Vernetzung der Matrix, zum anderen aber auch zu einem kovalenten Einbau des Wirkstoffes in die Matrix aus der dieser nicht mehr freigesetzt werden kann. Somit ist eine Einarbeitung dieser Wirkstoffe in die Mmatrix vor der Vernetzungsreaktion nicht möglich. Als Konsequenz müssen diese Substanzen in aufwendigen nachgeschalteten Prozessschritten in die Polyurethanmatrix eingebracht werden. Dies ist nicht nur für den Herstellungsschritt ein störender Umstand.

Aus dem Stand der Technik sind darüber hinaus eine Reihe von Schriften bekannt, in denen wirkstoffdotierte wasserabsorbierende Polymere offenbart werden. So beschreibt US 2003/0004479 eine wasserabsorbierende Zusammensetzung bestehend aus einem wasserabsorbierenden Polymer und einem pflanzlichen Pulver als Wirkstoff, wobei das wasserabsorbierende Polymer im Oberflächenbereich nachvernetzt ist.

Aufgabe der vorliegenden Erfindung ist es eine Haut- oder Wundauflage zur Verfügung zu stellen, die in der Lage ist Feuchtigkeit, insbesondere Wasser, zu absorbieren und einen wundheilungsfördernden und/oder hautpflegenden Wirkstoff abzugeben. Aufgabe der Vorliegenden Aufgabe ist demnach auch eine Auflage bereit zu stellen, die in der Lage ist die menschliche Haut zu Pflegen, deren Widerstandskraft zu erhöhen und/oder eine Wunde zu heilen.

Aufgabe der vorliegenden Erfindung ist es insbesondere einen Wundverband zur Verfügung zu stellen, der in der Lage ist, Wundexudat aufzusaugen, der ausreichend Feuchtigkeit von der Haut aufsaugen und gegebenenfalls durch das Pflaster nach außen transportieren kann, der ein feuchtes Wundheilungsmilieu erzeugt, der hautverträglich ist, der schmerzfrei wiederablösbar ist und welcher wundheilungsfördernde und/oder hautpflegende Zusätze enthält, die über einen längeren Zeitraum kontrolliert abgegeben werden können.

Eine weitere Aufgabe ist es, eine Auflage bereit zu stellen, die durch den Zusatz wundheilungsfördernder und/oder hautpflegender Substanzen keinen Einfluss auf die gegebenenfalls selbstklebenden Eigenschaften der Auflage ausübt und der einfach und kostengünstig in der Herstellung ist.

Gelöst wird dieses Bündel an Aufgaben mit einer Auflage entsprechend den Hauptansprüchen. Gegenstand der Unteransprüche sind vorteilhafte Ausführungsformen der erfindungsgemäßen Auflage sowie dessen Verfahren zur Herstellung. Es war überraschend und für den Fachmann nicht vorauszusehen, dass eine Haut- oder Wundauflage beinhaltend

a. eine Polykondensatmatrix, bevorzugt eine Polyurethanmatrix, basierend auf mindestens einem Polykondensatmonomer mit mindestens einer Polykondensatgruppe, und
b. ein teilchenförmiges wasserabsorbierendes Polymer, beinhaltend mindestens eine wundheilungsfördernde und/oder mindestens eine hautpflegende Substanz, mit mindestens einer funktionellen Gruppe, die mit der Polykondensatgruppe unter Bildung einer kovalenten Bindung reagieren könnte, oder
c. ein wasserabsorbierendes Polymer enthaltend mindestens eine wundheilungsfördemde und/oder mindestens eine hautpflegende Substanz,

wobei das teilchenförmige wasserabsorbierende Polymer mindestens teilweise von der Polykondensatmatrix umgeben ist,
wobei mindestens das teilchenförmige wasserabsorbierende Polymer die Wundheil- und/oder Hautpflegesubstanz aufweist,
wobei die Haut- oder Wundauflage eine Wundheilsubstanz- oder Wirkstoffverfügbarkeit von mindestens 10 Gew.% nach dem hierin angegebenen Extraktions-Test zeigt, und
wobei die wundheilungsfördemde und/oder hautpflegende Substanz in den wasserabsorbierenden Polymer inkorporiert ist, die Aufgaben löst.

Insbesondere löst eine Haut- oder Wundauflage, umfassend eine luft- und wasserdampfdurchlässige, bevorzugt selbstklebende Polyurethan-Matrix, enthaltend ein wasserabsorbierendes Polymer in das mindestens eine wundheilungsfördernde und/oder mindestens eine hautpflegende Substanz, nachfolgend auch als Wirkstoff bezeichnet, inkorporiert ist, die gestellten Aufgaben und hilft den Nachteilen des Standes der Technik ab.

Unter "wasserabsorbierend" wird erfindungsgemäß neben der Fähigkeit eines Stoffes Wasser in sich unter Bildung eines Hydrogels aufzunehmen jegliche Aufnahme von wässrigen Flüssigkeiten, insbesondere wässrige Körperflüssigkeiten wie Urin, Blut, Blutbestandteile wie Eiter, Lymphflüssigkeiten oder Blutserum, verstanden.

Als Polykondensate werden bevorzugt Polyurethane erfindungsgemäß eingesetzt. In der Regel werden Polyurethane aus den bekannten Ausgangsverbindungen der Polyurethanchemie nach bekannten Verfahren hergestellt, die in den Patenten DE-OS 3103499, DE-OS 3103500, EP 0 147 588 A1, EP 0 665 856 B1 oder DE 196 18 825 A1 dargestellt werden.
Polyurethan wird als Grundlage für die wirkstoffhaltige Matrix verwendet. Die Herstellung des Polyurethans (c) erfolgt durch die Polymerisation eines Alkohols (a) mit einem Isocyanat (b).

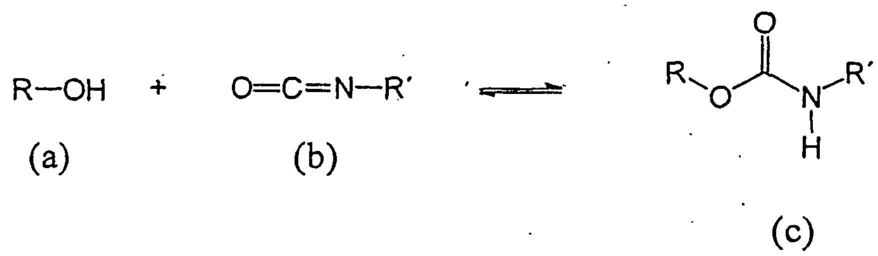

Ein entscheidender Vorteil der Polyurethanpolymer- oder gelmatrizes sind ihre selbstklebenden Eigenschaften, die ein zusätzliches Aufbringen einer Adhäsionsschicht auf die Matrix zur Fixierung des Wundverbandes im Bereich der Haut überflüssig machen. Im einfachsten Fall befindet sich die wirkstoffhaltige Polyurethanmatrix zwischen einer mit ihr fest verankerten Abdeckschicht, auch als Trägerschicht benannt, und einer abziehbaren Trennschicht.
Die abziehbare Trennschicht dient zur Sicherung der Klebeschicht, zur Verbesserung der Transport- und Lagerstabilität und wird vor dem Applizieren auf die Haut entfernt. Die Polyurethanmatrix kann auf einer Trägerschicht oder -folie aufgebracht sein, wie sie aus dem Stand der Technik bekannt ist. Die Trägerfolie besteht aus einer luft- und wasserdampfdurchlässigen aber wasserundurchlässigen Polymerschicht mit einer Dicke von ca. 10 bis 100 μm. Die u.U. flexible Trägerfolie besteht vorzugsweise aus Polymeren von Polyurethan, PE, PP, Polyamid, Polyester oder Polyether-ester. Ist die Polyurthanmatrix nicht selbstklebend ausgeführt

Geeignete Polyurethane als Matrix sind Gegenstand der DE 196 18 825, in der hydrophile, selbstklebende Polyurethangele offenbart werden, die bestehen aus

a) 2 bis 6 Hydroxylgruppen aufweisenden Polyetherpolyolen mit OH-Zahlen von 20 bis 112 und einem Ethylenoxid (EO)-Gehalt von ≥ 10 Gew.-%,
b) Antioxidantien,
c) in den Polyolen a) löslichen Wismut-(III)-Carboxylaten auf Basis von Carbonsäuren mit 2 bis 18 C-Atomen als Katalysatoren sowie
d) Hexamethylendiisocyanat,

mit einem Produkt der Funktionalitäten der Polyurethan bildenden Komponenten a) und d) von mindestens 5,2, wobei die Katalysatormenge c) 0,005 bis 0,25 Gew.-%, bezogen auf das Polyol a) beträgt, die Menge an Antioxidantien b) im Bereich von 0,1 bis 1,0 Gew.-%, bezogen auf Polyol a) liegt und ein Verhältnis von freien NCO-Gruppen der Komponente d) zu den freien OH-Gruppen der Komponente a) (Isocyanatkennzahl) im Bereich von 0,30 bis 0,70 gewählt wird.

Es werden bevorzugt 3 bis 4, ganz besonders bevorzugt 4-Hydroxylgruppen aufweisende Polyetherpolyole eingesetzt mit einer OH-Zahl im Bereich von 20 bis 112, bevorzugt 30 bis 56. Der Ethylenoxidgehalt liegt bei den erfindungsgemäß eingesetzten Polyetherpolyolen bei vorzugsweise ≥ 20 Gew.-%.

Die Polyetherpolyole sind als solche an sich bekannt und werden zum Beispiel durch Polymerisation von Epoxiden, wie Ethylenoxid, Propylenoxid, Butylenoxid oder Tetrahydrofuran, mit sich selbst oder durch Anlagerung dieser Epoxide, vorzugsweise von Ethylenoxid und Propylenoxid - gegebenenfalls im Gemisch untereinander oder separat nacheinander - an Starterkomponenten mit mindestens zwei reaktionsfähigen Wasserstoffatomen, wie Wasser, Ethylenglykol, Propylenglykol, Diethylenglykol, Dipropylenglykol, Glyzerin, Trimethylolpropan, Pentaerythrit, Sorbit oder Succrose, hergestellt. Vertreter der genannten, zu verwendenden höhermolekularen Polyhydroxylverbindungen sind zum Beispiel in High Polymers, Vol. XVI, "Polyurethanes, Chemistry and Technology" (Saunders-Frisch, Interscience Publishers, New York, Bd 1, 1962, S. 32-42) aufgeführt.

Als Isocyanatkomponente wird monomeres oder trimerisiertes Hexamethylendiisocyanat oder durch Biuret-, Uretdion-, Allophanatgruppen oder durch Prepolymerisierung mit Polyetherpolyolen oder Mischungen von Polyetherpolyolen auf Basis der bekannten Starterkomponenten mit 2 oder > 2 reaktionsfähigen H-Atomen und Epoxiden, wie Ethylenoxid oder Propylenoxid einer OH-Zahl von ≤ 850, bevorzugt 100 bis 600, modifiziertes Hexamethylendiisocyanat eingesetzt. Bevorzugt ist der Einsatz von modifiziertem Hexamethylendiisocyanat, insbesondere durch Prepolymerisierung mit Polyetherdiolen der OH-Zahl 200 bis 600 modifiziertes Hexamethylendiisocyanat. Ganz besonders bevorzugt sind Modifizierungen des Hexamethylendiisocyanats mit Polyetherdiolen der OH-Zahl 200-600, deren Restgehalt an monomeren Hexamethylendiisocyanat unter 0,5 Gew.-% liegt.

Als Katalysatoren kommen für die erfindungsgemäßen Polyurethangele in den wasserfreien Polyetherpolyolen a) lösliche Wismut(III)-Carboxylate auf Basis linearer, verzweigter, gesättigter oder ungesättigter Carbonsäuren mit 2 bis 18, vorzugsweise 6 bis 18 C-Atomen in Frage. Bevorzugt sind Bi(III)Salze verzweigter gesättigter Carbonsäuren mit tertiären Carboxylgruppen, wie der 2,2-Dimethyl- Octansäure (zum Beispiel Versatic-Säuren, Shell). Gut geeignet sind Zubereitungen dieser Bi(III)Salze in überschüssigen Anteilen dieser Carbonsäuren. Hervorragend bewährt hat sich eine Lösung von 1 mol des Bi(III)Salzes der Versatic 10-Säure (2,2-Dimethyloctansäure) in einem Überschuß von 3 mol dieser Säure mit einem Bi-Gehalt von ca. 17%.
Es werden die Katalysatoren bevorzugt in Mengen von 0,03 bis 0,3 Gew.-%, bezogen auf das Polyol a), eingesetzt.

Als Antioxidantien kommen für die erfindungsgemäßen Polyurethangele insbesondere sterisch gehinderte phenolische Stabilisatoren, wie BHT (2,6-Di-tert.butyl-4-methylphenol), Vulkanox BKF (2,2 min -Methylen-bis-(6-tert.-butyl-4-methyl phenol) (Bayer AG), Irganox 1010 (Pentaerythrityl-tetrakis-[3-(3,5-ditert.-butyl-4- hydroxyphenyl)-propionat]), Irganox 1076 (Octadecyl-3-(3,5-ditert.-butyl-4- hydroxyphenyl)-propionat) (Ciba-Geigy) oder Tocopherol (Vitamin E) in Betracht. Bevorzugt werden solche vom Typ des α-Tocopherol eingesetzt. Die Antioxidantien werden bevorzugt in Mengen von 0,15 bis 0,5 Gew.-%, bezogen auf das Polyol a), eingesetzt.

Die Isocyanatkennzahl (Verhältnis der bei der Reaktion eingesetzten freien NCO-Gruppen zu den freien OH-Gruppen) der erfindungsgemäßen Polyurethangelmassen liegt je nach der Funktionalität der eingesetzten Isocyanat- und Polyolkomponenten im Bereich von 0,30 bis 0,70, bevorzugt im Bereich von 0,45 bis 0,60. Die für eine Gelbildung erforderliche Isocyanatkennzahl kann sehr einfach nach der folgenden Formel abgeschätzt werden:

$$f_{(Polyol)} \bullet (f_{(Isocyanat)} - 1) \bullet Kennzahl \approx 2$$

$$Kennzahl \approx \frac{2}{f_{(Polyol)} \bullet (f_{(Isocyanat)} - 1)}$$

f: Funktionalität der Isocyanat- oder Polyolkomponente

Je nach angestrebter Klebrigkeit oder Elastizität des Gels kann die tatsächlich zu verwendende Isocyanatkennzahl um bis zu ± 20% von dem berechneten Wert abweichen. Die erfindungsgemäßen Polyurethangelmassen werden hergestellt nach üblichen Verfahren, wie sie beispielsweise beschrieben sind in Becker/Braun, Kunststoff- Handbuch, Bd. 7, Polyurethane, S. 121 ff, Carl-Hauser, 1983.

Weiter vorzugsweise kommen Polyurethangele zum Einsatz, wie sie in der EP 0 665 856 B1 offenbart sind. Die hydrophilen Polyurethane sind demnach erhältlich aus

1. einem Polyurethangel, welches

(A) 25-62 Gew.-%, vorzugsweise 30-60 Gew.-%, besonders bevorzugt 40-57 Gew.-%, bezogen auf die Summe aus (A) und (B), eines kovalent vernetzten Polyurethans als hochmolekulare Matrix und
(B) 75-38 Gew.-%, vorzugsweise 70-40 Gew.-%, besonders bevorzugt 60-43 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls
(C) 0 bis 100 Gew.-%, bezogen auf die Summe aus (A) und (B), an Füll- und/oder Zusatzstoffen enthält,

und welches erhältlich ist durch Umsetzung einer Mischung von

a) einem oder mehreren Polyisocyanaten,
b) einer oder mehreren Polyhydroxylverbindungen mit einem mittleren Molekulargewicht zwischen 1000 und 12000, und einer mittleren OH-Zahl zwischen 20 und 112,
c) gegebenenfalls Katalysatoren oder Beschleunigern für die Reaktion zwischen isocyanat- und Hydroxylgruppen sowie gegebenenfalls
d) aus der Polyurethanchemie an sich bekannten Füll- und Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_p$) zwischen 3 und 6 beträgt und die Isocyanatkennzahl (K) der Formel

$$K = \frac{300 \pm X}{(F_I \bullet F_p) - 1} + 7$$

gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Molekulargewicht und OH-Zahl als Zahlenmittel zu verstehen sind,
2. einem Wasser absorbierenden Material und/oder
3. einem nichtwässrigen Schäumungsmittel.

Bei der Herstellung von bevorzugt selbstklebenden Polyurethanen ist zu berücksichtigen, dass bei der Auswahl

der gelbildenden Komponenten die oben definierten Bedingungen eingehalten werden, da sonst anstelle von selbsthaftenden Gelen klebfreie, elastische Gele erhalten werden.

Bevorzugte Polyhydroxylverbindungen sind Polyetherpolyole, wie sie in den oben genannten Offenlegungsschriften ausführlich genannt sind.

Als Polyisocyanatkomponenten sind sowohl (cyclo)aliphatische als auch aromatische Isocyanate geeignet. Bevorzugte (cyclo)aliphatische Polyisocyanate sind 1,6-Hexamethylen-diisocyanat sowie dessen Biurete und Trimerisate bzw. hydrierte Diphenylmethandiisocyanat ("MDI")-Typen. Bevorzugte aromatische Polyisocyanate sind solche, die durch Destillation erhalten werden, wie MDI-Gemische aus 4,4'- und 2,4'-Isomeren - oder 4,4'-MDI, sowie Toluylendiisocyanat ("TDI")-Typen.

Die Diisocyanate können insbesondere zum Beispiel aus der Gruppe der unmodifizierten aromatischen oder aliphatischen Diisocyanate oder aber aus durch Prepolymerisierung mit Aminen, Polyolen oder Polyetherpolyolen gebildeten modifizierten Produkten gewählt werden.

Als Vorteile der erfindungsgemäßen Polyurethane im Vergleich zu anderen Polykondensaten und Polymeren, die insbesondere für die Herstellung von Verbandsmaterialien verwendet werden, lassen sich folgende Punkte nennen:

- Polyurethan kann flexibel als selbstklebende oder nichtklebende Matrix bereit gestellt werden.
- als selbstklebendes System kann auf einen Zusatz weiterer Klebstoffe, die unter Umständen Nebenwirkungen wie Mazeration, Entzündungen der dermalen Bereiche, Reduktion der Hautatmung u.a. hervorrufen, verzichtet werden.
- Polyurethane erweisen sich gegenüber anderen Klebematerialen, wie Polyacrylate, Kautschuk etc., als äußerst vorteilhaft, da sie kein Allergiepotential beinhalten.
- Polyurethan weist eine sehr gute Wasserdampfdurchlässigkeit auf. Hierdurch ist gewährleistet, dass bei einer Applikation über einen längeren Zeitraum keine Mazeration durch die Wasserabgabe der Haut erfolgt.
- Die Sauerstoffdurchlässigkeit des Polyurethan sorgt für eine gute Versorgung der abdeckten Hautstelle mit Sauerstoff, wodurch einer Schädigung des Gewebes entgegengewirkt wird.
- Polyurethan ist allergieneutral, so dass nach der Applikation mit keiner allergischen Reaktionen des Organismus zu rechnen ist.
- Polyurethan zeigt zudem gegenüber anderen Materialien wie z.B. Hydrocolloiden oder Hydrogelen keine Neigung bei längerem Kontakt mit Flüssigkeiten wie Wundexudat zu desintegrieren. Ein aus Polyurethan hergestellter Wundverband hinterläßt demnach, bei längerem Kontakt mit Wundflüssigkeit, keine die weitere Wundheilung störenden Rückstände in der Wunde.
- Selbstklebend ausgerüstetes Polyurethan entklebt bei Kontakt mit Flüssigkeit, so dass ein Verkleben mit frisch gebildetem Gewebe vermieden wird und zudem eine schmerzfreie Ablösung des Wundabdeckung gewährleistet ist.
- Erfindungsgemäße Polyurethanwundauflagen erzeugen ein feuchtes Wundmilieu, was zu einer schnelleren Wundheilung führt.

Die Polymermatrix, bevorzugt die Polyurethanmatrix, kann partiell oder vollflächig geschäumt und/oder ungeschäumt, ungefüllt oder mit zusätzlichen Füllstoffen, wie beispielsweise Titandioxid, Zinkoxid, Weichmachern, Farbstoffen etc. eingesetzt werden.

Über ein Schäumen der Matrix kann eine bessere Polsterwirkung und damit verbunden eine verbesserte Haptik für den Anwender erreicht werden.

Die Matrix, insbesondere das Polyurethanpolymer, kann gegebenenfalls aus der Polyurethan-Chemie an sich bekannte Zusatzstoffe enthalten, wie zum Beispiel Füllstoffe und Kurzfasern auf anorganischer oder organischer Basis, Metallpigmente, oberflächenaktive Substanzen oder flüssige Streckmittel wie Substanzen mit einem Siedepunkt von über 150°C.

Als anorganische Füllstoffe seien beispielsweise Schwerspat, Kreide, Gips, Kieserit, Soda, Titandioxid, Ceroxid, Quarzsand, Kaolin, Russ und Mikrohohlkugeln genannt. An organischen Füllstoffen können zum Beispiel Pulver auf Basis von Polystyrol, Polyvinylchlorid, Harnstoff-Formaldehyd und Polyhydrazodicarbonamid eingesetzt werden. Als Kurzfasern kommen zum Beispiel Glasfasern von 0,1 - 1 mm Länge oder Fasern organischer Herkunft, wie zum Beispiel Polyester- oder Polyamidfasern, in Frage. Metallpulver, wie zum Beispiel Eisen-, Aluminium-, oder Kupferpulver, können ebenfalls bei der Gelbildung mitverwendet werden. Um den Gelen die gewünschte Färbung zu verleihen, können die bei der Einfärbung von Polyurethanen an sich bekannten Farbstoffe oder Farbpigmente auf organischer oder anorganischer Basis verwendet werden, wie zum Beispiel Eisenoxid- oder Chromoxidpigmente, Pigmente auf Phthalocyanin- oder Monoazo-Basis. Als oberflächenaktive Substanzen seien zum Beispiel Cellulosepulver, Aktivkohle und Kieselsäurepräparate genannt.

Zur Modifizierung der Hafteigenschaften der Gele können gegebenenfalls Zusätze von polymeren Vinylverbindungen, Polyacrylaten und sonstigen in der Klebstoff-Technik üblichen Copolymeren bzw. auch Klebemittel auf Naturstoffbasis bis zu einem Gehalt von 10 Gew.-%, bezogen auf das Gewicht der Gelmasse, zugegeben werden, ohne die vorteilhaften Eigenschaften der Polyurethane zu verändern.

Die Polymermatrix kann vorteilhafterweise transparent eingestellt werden. Als transparent, wasserdampfdurchlässig und klebend erfüllt die Matrix damit ästhetische und anwendungsfreundliche Aspekte. Dies stellt einen signifikanten vorteilhaften Unterschied zu den Polyacylat und Silikongel basierenden Pflastersystemen dar. Die Transparenz erhöht zudem die Akzeptanz beim Anwender, da die erfindungsgemäßen Haut- oder Wundauflagen, insbesondere als Pflaster, üblicherweise über einen längeren Zeitraum auf der Haut getragen werden können.

Ist die erfindungsgemäße Auflage selbstklebend ausgerüstet, kann auf zusätzliche Befestigungsmittelverzichtet werden. Die Wundauflage wird direkt als Verbandsmaterial auf die abzudeckende Wunde gelegt und haftet aufgrund der selbstklebenden Eigenschaften auf der die Wunde umgebenden Haut.

Bei größeren Wunden, wenn eine zusätzliche Verklebung erwünscht ist oder wenn die Polymermatrix nicht selbstklebend' ausgerüstet ist, kann durch Zusatz einer Randschichtverklebung die Wundauflage auf der Haut verklebt werden.

Das erfindungsgemäße Verbandsmaterial ist dann entsprechend bekannten Wundverbände aufgebaut. Sie bestehen im allgemeinen aus einem Trägermaterial, das auf einer Seite mit einer selbstklebenden Schicht versehen ist. Auf diese selbstklebende Beschichtung ist dann die erfindungsgemäße Wundauflage aufgebracht. Um eine einfache Handhabung zu gewährleisten, wird die selbstklebende Beschichtung darüber hinaus mit einer schützenden Schicht eingedeckt, zum Beispiel einem Siegelpapier.

Eine geeignete Klebemasse für die Randschichtverklebung über das zusätzliche Trägermaterial ist in der Schrift DE 27 43 979 C3 dargelegt, weiterhin sind. für die Klebebeschichtung bevorzugt handelsübliche druckempfindliche Klebmassen auf Acrylat- oder Kautschukbasis einsetzbar.

Besonders bevorzugt werden thermoplastische Heißschmelzklebemassen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich. Gegebenenfalls kann eine Nachvernetzung durch UV- oder Elektronenstrahlen-Bestrahlung angebracht sein. Insbesondere Heißschmelzkfebemassen auf Basis von Blockcopolymeren zeichnen sich durch ihre vielfältige Variationsmöglichkeiten aus, denn durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut auch an kritischen Stellen des menschlichen Bewegungsapparates gewährleistet.

Die hohe Scherfestigkeit der Heißschmelzklebemasse wird durch die hohe Kohäsivität des Polymeren erreicht. Die gute Anfaßklebrigkeit ergibt sich durch die eingesetzte Palette an Klebrigmachern und Weichmachern.

Die Klebemasse beinhaltet vorzugsweise mindestens eine aromatische Komponente, welche einen Anteil von weniger als 35 %, bevorzugt 5 bis 30 %, aufweist.

Für besonders starkklebende Systeme basiert die Heißschmelzklebemasse bevorzugt auf Blockcopolymeren, insbesondere A-B-, A-B-A-Blockcopolymere oder deren Mischungen. Die harte Phase A ist vornehmlich Polystyrol oder dessen Derivate, und die weiche Phase B enthält Ethylen, Propylen, Butylen, Butadien, Isopren oder deren Mischungen, hierbei besonders bevorzugt Ethylen und Butylen oder deren Mischungen. Insbesondere die gezielte Abmischung von Di-Block- und Tri-Blockcopolymeren ist vorteilhaft, wobei ein Anteil an Di-Blockcopolymeren von kleiner 80 Gew.% bevorzugt wird.

In einer vorteilhaften Ausführung weist die Heißschmelzklebemasse die nachfolgend angegebene Zusammensetzung auf:

| | |
|---|---|
| 10 Gew.% bis 90 Gew.% | Blockcopolymere, |
| 5 Gew.% bis 80 Gew.% | Klebrigmacher wie Öle, Wachse, Harze und/oder deren Mischungen, bevorzugt Mischungen- aus Harzen und Ölen, |
| weniger als 60 Gew.% | Weichmacher, |
| weniger als 15 Gew.% | Additive, |
| weniger als 5 Gew.% | Stabilisatoren. |

Die als Klebrigmacher dienenden aliphatischen oder aromatischen Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Hautverklebung auswirken. Als Weichmacher finden mittel- oder langkettige Fettsäuren und/oder deren Ester Verwendung. Diese Zusätze dienen dabei der Einstellung der Klebeeigenschaften und der Stabilität. Gegebenenfalls kommen weitere Stabilisatoren und andere Hilfsstoffe zum Einsatz.

Die Trägermaterialien bestehen bevorzugt aus einer luft- und wasserdampfdurchlässigen aber wasserundurchlässigen Polymerschicht mit einer Dicke von ca. 10 bis 100 μm. Die u.U. flexible Trägerfolie besteht vorzugsweise aus Polymeren von Polyurethan, PE, PP, Polyamid, Polyester oder Polyether-ester oder bekannten Trägermaterialien wie

Gewebe, Vliese, Schäume, Kunststoffe etc.
Die erfindungsgemäße Polyurethanmatrix kann auf dieser Trägerschicht oder -folie aufgebracht sein, wie sie aus dem Stand der Technik bekannt ist. Dabei wird die Matrix einseitig mit dem Trägermaterial abgedeckt und als Verbundfolie appliziert. , Je nach verwendetem Trägermaterial können dadurch die Wasserdampfdurchlässigkeit, die Festigkeit der Wundabdeckung, die Polsterung gegen Druck und andere physikalische Eigenschaften der Wundabdeckung gesteuert werden.

Ein erfindungsgemäß ausgestattetes Verbandsmaterial, mit oder ohne zusätzlicher Randverklebung, wird dann wie üblich auf die Wunde aufgelegt.

Das direkte Einbringen zahlreicher wundheilungsfördernder und/oder hautpflegender Substanzen in die Matrix, insbesondere in die Polyurethanmatrix, ist vor deren Vernetzungsreaktion nicht möglich, da diese Substanzen über aktive Wasserstoffatome (Hydroxyl-, Amino- oder Säuregruppen) verfügen, welche in der Vernetzungsreaktion, beispielsweise bei der Polyurethanbildung, mitreagieren würden. Die Folgen wären eine untervernetzte Matrix und kovalent gebundene, zur Wundheilung oder Hautpflege nicht mehr zur Verfügung stehende Wirkstoffe. Diesem Problem kann erfindungsgemäß. Abhilfe geschaffen werden, indem die wundheitungsfördernden oder hautpflegenden Substanzen, Wirkstoffe in verkapselter Form in das Reaktionsgemisch eingebracht und gleichzeitig der Vernetzungsreaktion entzogen werden.

Dazu werden die Substanzen mit Hilfe von wasserabsorbierenden Polymeren, wie beispielsweise Superabsorbern, eingebunden bzw. verkapselt was zusammenfassend als auch als inkorporieren bezeichnet wird.

Superabsorber, in denen Substanzen oder Wirkstoffe wie beispielsweise Dexpanthenol in verkapselter Form vorliegen, sind beispielsweise vernetzte Natriumpolyacrylate wie beispielsweise bekannt unter Favor T®. Sie bestehen aus einer vernetzten Polyacrylatmatrix, in welche der betreffende Wirkstoff je nach Wirkstofftyp vor oder nach der Polymerisation in die Matrix eingebracht wurde und aus dieser erst bei einem Quellvorgang wieder freigesetzt werden kann. Diese Produkte können, ohne die Vernetzungsreaktion der Polyurethanmatrix zu inhibieren, vor der Reaktion in die unvernetzten Polyurethanmatrixrohstoffe eingearbeitet werden. Der mit dem Wirkstoff dotierte Superabsorber gibt aus der vernetzten Matrix bei der Anwendung, d.h. beim Kontakt mit wässrigen Medien, wie beispielsweise dem Wundexudat, den Wirkstoff über einen längeren Zeitraum und vorteilhaft konstant dann erst wieder ab.
Bei dem erfindungsgemäßen Haut- oder Wundauflagen ist bevorzugt, dass das wasserabsorbierende Polymer umfasst

(α1) 0,1 bis 99,999 Gew.-%, bevorzugt 20 bis 98,99 Gew.% und besonders bevorzugt 30 bis 98,95 Gew.-% polymerisierte, ethylenisch ungesättigte, säuregruppenhaltige Monomere oder deren Salze oder polymerisierte, ethylenisch ungesättigte, einen protonierten oder quarternierten Stickstoff beinhaltende Monomere, oder deren Mischungen, wobei mindestens ethylenisch ungesättigte, säuregruppenhaltige Monomere, vorzugsweise Acrylsäure, beinhaltende Mischungen besonders bevorzugt sind,
(α2) 0 bis 70 Gew.-%, bevorzugt 1 bis 60 Gew.-% und besonders bevorzugt 1 bis 40 Gew.-% polymerisierte, ethylenisch ungesättigte, mit (α1) copolymerisierbare Monomere,
(α3) 0,001 bis 10 Gew.-%, bevorzugt 0,01 bis 7 Gew.-% und besonders bevorzugt 0,05 bis 5 Gew.-% eines oder mehrerer Vernetzer,
(α4) 0 bis 30 Gew.-%, bevorzugt 1 bis 20 Gew.-% und besonders bevorzugt 5 bis 10 Gew.-% wasserlösliche Polymere, sowie
(α5) 0 bis 20 Gew.-%, bevorzugt 0,01 bis 7 Gew.-% und besonders bevorzugt 0,05 bis 5 Gew.-% eines oder mehrere Hilfsstoffe,

wobei die Summe der Gewichtsmengen (α1) bis (α5) 100 Gew.-% beträgt.

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 50 Mol-% und darüber hinaus bevorzugt zu 50-90 Mol-% neutralisiert. Die Neutralisation der Monomere (α1) kann vor und auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Bevorzugte monoethylenisch ungesättigte, säuregruppenhaltige Monomere (α1) sind Acrylsäure, Methacrylsäure, Ethacrylsäure, α-Chloracrylsäure, α-Cyanoacrylsäure, β-Methylacrylsäure (Crotonsäure), α-Phenylacrylsäure, β-Acryloxypropionsäure, Sorbinsäure, α-Chlorsorbinsäure, 2'-Methylisocrotonsäure, Zimtsäure, p-Chlorzimtsäure, β-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als monoethylenisch ungesättigte, säuregruppen-

haltige Monomere ($\alpha$1) des Weiteren ethylenisch ungesättigte Sulfonsäuremonomere oder ethylenisch ungesättigte Phosphonsäuremonomere bevorzugt.

Ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Stryrolsulfonsäure bevorzugt. Als Acryl- bzw. Methacrylsulfonsäuren sind Sulfoethyl(meth)acrylat, Sulfopropyl(meth)acrylat und, 2-Hydroxy-3-methacryloxypropylsulfonsäure. Als (Meth)Acrylamidoalkylsulfonsäure ist 2-Acrylamido-2-methylpropansulfonsäure bevorzugt.

Ferner sind ethylenisch ungesättigte Phosphonsäuremonomere, wie Vinylphosphonsäure, Allylphosphonsäure, Vinylbenzylphosponsäure, (Meth)acrylamidoalkylphosphonsäuren, Acrylamidoalkyldiphosphonsäuren, phosponomethylierte Vinylamine und (Meth)acrylphosphonsäurederivate bevorzugt.

Es ist erfindungsgemäß bevorzugt, dass das wasserabsorbierende Polymer zu mindestens 50 Gew.-%, vorzugsweise zu mindestens 70 Gew.-% und darüber hinaus bevorzugt zu mindestens 90 Gew.-% auf carboxylatgruppenhaltigen Monomeren besteht. Es ist erfindungsgemäß besonders bevorzugt, dass das wasserabsorbierende Polymer zu mindestens 50 Gew.-%, vorzugsweise zu mindestens 70 Gew.-% aus Acrylsäure besteht, die vorzugsweise zu mindestens 20 Mol-%, besonders bevorzugt zu mindestens 50 Mol-% und darüber hinaus bevorzugt im Bereich von 65 bis 85 Mol-%, vorzugsweise mit Natronlauge, neutralisiert ist.

Als ethylenisch ungesättigte, einen protonierten Stickstoff enthaltende Monomere ($\alpha$1) sind vorzugsweise Dialkylaminoalkyl(meth)acrylate in protonierter Form, beispielsweise Dimethylaminoethyl(meth)acrylat-Hydrochlorid oder Dimethylaminoethyl(meth)acrylat-Hydrosulfat, sowie Dialkylaminoalkyl-(meth)acrylamide in protonierter Form, beispielsweise Dimethylaminoethyl(meth)-acrylamid-Hydrochlorid, Diemethylaminopropyl(meth)acrylamid-Hydrochlorid, Dimethylaminopropyl(meth)acrylamid-Hydrosulfat oder Dimethylaminoethyl(meth)-acrylamid-Hydrosulfat bevorzugt.

Als ethylenisch ungesättigte, einen quarternierten Stickstoff enthaltende Monomere ($\alpha$1) sind Dialkylammoniumalkyl(meth)acrylate in quarternisierter Form, beispielsweise Trimethylammoniumethyl(meth)acrylat-Methosulfat oder Dimethylethylammoniumethyl(meth)acrylat-Ethosulfat sowie (Meth)acrylamido-alkyldialkylamine in quarternisierter Form, beispielsweise (Meth)acrylamidopropyl-trimethylammoniumchlorid, Tremethylammoniumethyl(meth)acrylat-Chlorid oder (Meth)acrylamidopropyltrimethylammoniumsulfat bevorzugt.

Als monoethylenisch ungesättigte, mit ($\alpha$1) copolymerisierbare Monomere ($\alpha$2) sind Acrylamide und Methacrylamide bevorzugt.

Mögliche (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino(meth)acrylamid, Dimethyl(meth)acrylamid oder Diethyl(meth)acrylamid Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Des Weiteren sind als monoethylenisch ungesättigte, mit ($\alpha$1) copolymerisierbaren Monomere ($\alpha$2) in Wasser dispergierbare Monomere bevorzugt. Als in Wasser dispergierbare Monomere sind Acrylsäureester und Methacrylsäurester, wie Methyl(meth)acrylat, Ethyl(meth)acrylat, Propyl(meth)acrylat oder Butyl(meth)acrylat, sowie Vinylacetat, Styrol und Isobutylen bevorzugt.

Erfindungsgemäß bevorzugte Vernetzer ($\alpha$3) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren ($\alpha$1) oder ($\alpha$2) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren ($\alpha$1) oder ($\alpha$2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV). Dabei wird durch die Verbindungen der Vernetzerklasse I eine Vernetzung der Polymere durch die radikalische Polymerisation der ethylenisch ungesättigten Gruppen des Vernetzermoleküls mit den monoethylenisch ungesättigten Monomeren ($\alpha$1) oder ($\alpha$2) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere ($\alpha$1) oder ($\alpha$2) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren ($\alpha$1) oder ($\alpha$2).

Bevorzugte Verbindungen der Vernetzerklasse I sind Poly(meth)acrylsäureester, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth) allyverbindungen, (Meth)acrylsäureester einer Monovinylverbindung oder (Meth)acrylsäureester einer Mono(meth)allylverbindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen. Die Offenbarungen werden hiermit als Referenz eingeführt und gelten somit als Teil der Offenbarung.

Als Verbindungen der Vernetzerklasse I seien als Beispiel genannt Alkenyldi(meth)acrylate, beispielsweise Ethylenglykoldi(meth)acrylat, 1,3-Propylenglykoldi(meth)acrylat, 1,4-Butylenglykoldi(meth)acrylat, 1,3-Butylenglykoldi (meth)acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopentandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'-(1,2-Dihydroxyethylen)bis(meth)acrylamid, N,N'-Hexamethylenbis(meth)acrylamid oder N,N'-Methylenbis(meth)acrylamid, Polyalkoxydi(meth)acrylate, beispielsweise Diethylenglykoldi(meth)acrylat, Triethylenglykoldi(meth)acrylat, Tetraethylenglykoldi(meth)acrylat, Dipropylenglykoldi(meth)acrylat, Tripropylenglykoldi(meth)acrylat oder Tetrapropylenglykoldi(meth)acrylat, Bisphenol-A-di(meth)acrylat, ethoxyliertes Bisphenol-A-di (meth)acrylat, Benzylidindi(meth)acrylat, 1,3-Di(meth)acryloyloxy-propanol-2, Hydrochinondi(meth)acrylat, Di(meth) acrylatester des vorzugsweise mit 1 bis 30 Mol Alkylenoxid pro Hydroxylgruppe oxyalkylierten, vorzugsweise ethoxylierten Trimethylolpropans, Thioethylenglykoldi(meth)acrylat, Thiopropylenglykoldi(meth)acrylat, Thiopolyethylenglykoldi(meth)acrylat, Thiopolypropylenglykoldi(meth)acrylat, Divinylether, beispielsweise 1,4-Butandioldivinylether, Divinylester, beispielsweise Divinyladipat, Alkandiene, beispielsweise Butadien oder 1,6-Hexadien, Divinylbenzol, Di(meth)allylverbindungen, beispielsweise Di(meth)allylphthatat oder Di(meth)allylsuccinat, Homo- und Copolymere von Di(meth) allyldimethylammoniumchlorid und Homo- und Copolymere von Diethyl(meth)allylaminomethyl(meth)acrylatammoniumchlorid, Vinyl-(meth)acrylVerbindungen, beispielsweise Vinyl(meth)acrylat, (Meth)allyl-(meth)acryl-Verbindungen, beispielsweise (Meth)allyl(meth)acrylat, mit 1 bis 30 Mol Ethylenoxid pro Hydroxylgruppe ethoxyliertes (Meth) allyl(meth) acrylat, Di(meth)allylester von Polycarbonsäuren, beispielsweise Di(meth)allylmaleat, Di(meth)allyfumarat, Di(meth)allylsuccinat oder Di(meth)allylterephthalat, Verbindungen mit 3 oder mehr ethylenisch ungesättigten, radikalisch polymerisierbaren Gruppen wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi(meth)acrylat, 3-Allyloxy-1,2-propandioldi(meth)acrylat, Tri(meth)allylcyanurat, Tri(meth)allylisocyanurat, Pentaerythrittetra(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri(meth)allylamin, Di(meth)allylalkylamine, beispielsweise Di(meth) allylmethylamin, Tri(meth)allylphosphat Tetra(meth)allylethylendiamin, Poly(meth)allylester, Tetra(meth)allyloxiethan oder Tetra(meth)allylammoniumhalide. Erfindungsgemäß sind in der Vernetzerklasse I vinylisocyanate, Trivinyltrimellitat oder Tri(meth)allylisocyanurat bevorzugt, wobei Trivinyltrimellitat besonders bevorzugt sind.

Als Verbindung der Vernetzerklasse II sind Verbindungen bevorzugt, die mindestens zwei funktionelle Gruppen aufweisen, die in einer Kondensationsreaktion (=Kondensationsvernetzer), in einer Additionsreaktion oder in einer Ringöffnungsreaktion mit den funktionellen Gruppen der Monomere ($\alpha$1) oder ($\alpha$2), bevorzugt mit Säuregruppen, der Monomeren ($\alpha$1), reagieren können. Bei diesen funktionellen Gruppen der Verbindungen der Vernetzerklasse II handelt es sich vorzugsweise um Alkohol-, Amin-, Aldehyd-, Glycidyl-, Isocyanat-, Carbonat- oder Epichlorfunktionen.

Als Verbindung der Vernetzerklasse II seien als Beispiele genannt Polyole, beispielsweise Ethylenglykol, Polethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin,' Diethanolamin, Triethanolamin oder Propanolamin, Polyaminverbindungen, beispielsweise Ethylendiamin, Diethylentriaamin, Triethylentetraamin, Tetraethylenpentaamin oder Pentaethylenhexaamin, Polyglycidylether-Verbindungen wie Ethylenglykoldiglycidylether, Polyethylenglykoldiglycidylether, Glycerindiglycidylether, Glycerinpolyglycidylether., Pentareritritpolyglycidylether, Propylenglykoldiglycidylether Polypropylenglykoldiglycidylether, Neopentylglykoldiglycidylether, Hexandiolglycidylether, Trimethylolpropanpolyglycidylether, Sorbitolpolyglycidylether, Phtahlsäurediglycidylester, Adipinsäurediglycidylether, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbutanol-tris[3-(1-aziridinyl)propionat], 1,6-Hexamethylendiethylenharnstoff und Diphenylmethan-bis-4,4'-N,N'-diethylenharnstoff, Halogenepoxide beispielsweise Epichlor- und Epibromhydrin und $\alpha$-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-,dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin.

Als Verbindungen der Vernetzerklasse II sind des weiteren Polyoxazoline wie 1,2-Ethylenbisoxazolin, Vernetzer mit Silangruppen wie γ-Glycidoxypropyltrimethoxysilan und γ-Aminopropyltrimethoxysilan, Oxazolidinone wie 2-Oxazolidinon, Bis- und Poly-2-oxazolidinone und Diglykolsilikate bevorzugt.

Als Verbindungen der Klasse III sind hydroxyl- oder aminogruppenhaltige Ester der (Meth)acrylsäure, wie beispielsweise 2-Hydroxyethyl(meth)acrylat, sowie hydroxyl- oder aminogruppenhaltige (Meth)acrylamide, oder Mono (meth)allylverbindungen von Diolen bevorzugt.

Die polyvalenten Metallkationen der Vernetzerklasse IV leiten sich vorzugsweise von ein- oder mehrwertigen Kationen ab, die einwertigen insbesondere von Alkalimetallen, wie Kalium, Natrium, Lithium, wobei Lithium bevorzugt wird. Bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. $AlCl_3 \times 6H_2O$, $NaAl(SO_4)_2 \times 12\ H_2O$, $KAl(SO_4)_2 \times 12\ H_2O$ oder $Al_2(SO_4)_3 \times 14\text{-}18\ H_2O$ eingesetzt.

Besonders bevorzugt werden $Al_2(SO_4)_3$ und seine Hydrate als Vernetzer der Vernetzungsklasse IV verwendet.

Bevorzugte sind wasserabsorbierende Polymere, die durch Vernetzer der folgenden Vernetzerklassen bzw. durch Vernetzer der folgenden Kombinationen von Vernetzerklassen vernetzt sind: I, II, III, IV, I II, I III, I IV, I II III, I II IV, I III IV, II III IV, II IV oder III IV. Die vorstehenden Kombinationen von Vernetzerklassen stellen jeweils für sich eine bevorzugte Ausführungsform von Vernetzern eines wasserabsorbierenden Polymers dar.

Einer weiteren bevorzugten Ausführungsform entsprechen wasserabsorbierende Polymere, die durch einen beliebigen der vorstehend genannten Vernetzer der Vernetzerklassen 1 vernetzt sind. Unter diesen sind wasserlösliche Vernetzer bevorzugt. In diesem Zusammenhang sind N,N'-Methylenbisacrylamid, Polyethylenglykotdi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den erfindungsgemäßen absorbierende Polymeren wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsstoffe (α5) werden vorzugsweise Stellmittel, Geruchsbinder, oberflächenaktive Mittel oder Antioxidatien eingesetzt.

Aus den vorgenannten Monomeren und Vernetzern lässt sich das wasserabsorbierende Polymer durch verschiedene Polymerisationsweisen herstellen, die dem Fachmann bekannt sind.

Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818. Die Offenbarungen werden hiermit als Referenz eingeführt und gelten somit als Teil der Offenbarung. Im Falle der vorstehend genannten Polymerisationsarten ist es bevorzugt, die Wundheilsubstanz bereits mit dem oder den in dem entsprechenden Polymerisationsverfahren eingesetzten Lösemittel oder Monomer bzw. Monomeren in den zuvor bereits beschriebenen Varianten als Gemisch in die Polymerisation einzuführen.

Vorzugsweise wird unter "Wundheilsubstanz" erfindungsgemäß eine Substanz oder eine Mischung aus Substanzen verstanden, wobei die Substanz oder mindestens eine Substanz der Mischung als funktionelle Gruppe eine Doppelbindung, eine OH-Gruppe, eine NH-Gruppe oder eine COOH-Gruppe oder ein Salz mindestens einer dieser Gruppen, vorzugsweise eine OH-Gruppe aufweist. Zudem ist es bevorzugt, dass die Wundheilsubstanz eine 2 bis 100 Kohlenstoffatome und ein bis 20 Sauerstoffatome aufweist. Die vorstehenden Eigenschaften sind ebenfalls für die erfindungsgemäßen Wirkstoffe oder Arzneimittelwirkstoffe bevorzugt.

Im allgemeinen werden als auf Pflanzenextrakten basierende Wundheilsubstanzen Equisetum arvense, Aloe barbadensis, Arnica montana, Arnica chamissonis, Symphytum officinale, Solanum dulcamara, Echinacea pallida, Potentilla erecta, Trigonella foenumgraecum, Juglans regia, Linum usitatissimum, Terminalia sericea, Oenothera biennis, Centella asiatica, Arctium lappa, Capsella bursa-pastoris, Hypericum perforatum, Matricaria recutita, Chamomille recutita, Agrimonia eupatoria, Centaurea cyanus, Larrea tridentata, Populus spec., Echinacea pupurea, Calendula officinalis, Aesculus hippocastanum, Salvia officinalis, Plantago lanceolata, Quercus robur, Glycyrhiza glabra, Quercus petraea, Hamamelis virgian, Cardiospermum halicacabum,Betula, Urtica dioica, Buxus chinensis, Lavandula angustifolia, Lavandula hybrida, Crocus sativus, Smilax aspera, Melaleuca alternifolia, Aminosäuren oder Viola tricolor oder deren Salze oder Derivate oder Mischungen aus mindestens zwei davon erfindungsgemäß eingesetzt.

Ferner kommen als weitere Wundheilsubstanzen oder Hautpflegemittel Vitamine und der gleichen wie Glukosamin Sulfate Allantoin, Biotin, Chondroitin Sulfate, Coenzym Q10, Dexpanthenol, Honig/Honigextrakt, Niacinamid, Propolis, Vitamin A oder seine Ester, Vitamin C und seine Ester, Vitamin E und seine Ester oder deren Salze oder Derivate oder Mischungen aus mindestens zwei davon erfindungsgemäß in Betracht.

Erfindungsgemäß bevorzugt handelt es sich bei Wundheilsubstanzen um Dexpanthenol oder Extrakte der Ringelblume, vorzugsweise Calendulaöl; der Hamamelis, vorzugsweise D-Hamelose; oder der Kamille, vorzugsweise das Öl der Kamillenblütevorzugsweise Bisbolol oder Azulen - oder Mischungen aus mindestens zwei aller vorstehenden Substanzen.

Weiterhin ist es bevorzugt, dass jeweils eine der vorstehenden Wundheilsubstanzen in einer Mischung als Hauptkomponente vorliegen kann, wobei diese Hauptkomponente vorzugsweise mindestens 50 Gew.-%, vorzugsweise mindestens 70 Gew.-% und besonders bevorzugt mindestens 95 Gew.-%, jeweils bezogen auf die Mischung, vorliegen kann.

Als hautpflegende Substanzen werden bevorzugt Vitamine, Antioxidantien, Lichtschutzmittel, Insektenrepellentien, ätherische Öle, antimikrobielle Mittel, Moisturizer, Parfume und insbesonder Coenzym Q10 gewählt.

Die wundheilungsfördernden und/oder hautpflegenden Substanzen, die einzeln oder vermischt eingesetzt werden können, sind vorteilhaft zu 0,1 bis 10,0 Gew.%, bevorzugt 0,2 bis 5 Gew.%, bezogen auf die Polymermatrix, bzw. zu 0,001 bis 30 Gew.%, bevorzugt 5 bis 15 Gew.%, bezogen auf das wirkstoffdotierte wasserabsorbierende Polymer, enthalten.

Die Einarbeitung der Wundheil- oder Hautpflegesubstanz in das wasserabsorbierende Polymer vor Abschluss der Bildung, bzw. vor Beginn der Polymerisation des wasserabsorbierenden Polymers kann durch folgende Verfahrensschritte erfolgen. Zum einen kann die Substanz über das zur Herstellung des wasserabsorbierenden Polymers verwendeten Lösemittel in das wasserabsorbierende Polymer eingearbeitet werden. Zum anderen kann die Substanz dem zur Bildung des wasserabsorbierenden Polymer verwendeten Monomer, Oligomer oder Präpolymer oder mindestens zwei davon zugesetzt. In beiden vorstehenden Varianten kann die Wundheil- oder Hautpflegesubstanz als Lösung, Emulsion oder Suspension vorliegen. Weiterhin können die beiden vorstehenden Varianten miteinander kombiniert werden.

In einer anderen Ausführungsform des erfindungsgemäßen Verfahren wird die Substanz nach Abschluss der, Bildung des wasserabsorbierenden Polymers oder während dessen Weiterverarbeitung oder beides eingearbeitet. Diese Einarbeitung erfolgt vorzugsweise in ein Gel des wasserabsorbierenden Polymers. Hierbei ist es bevorzugt, dass das Gel eine auf das wasserabsorbierenden Polymer bezogene Wassermenge vom 0,2- bis 20-fachen, vorzugsweise 1- bis 10-fachen und besonders bevorzugt 2- bis 4-fachen aufweist, um eine möglichst gleichmäßige Einarbeitung der Wundheilsubstanz zu erreichen.

Dieses kann zum einen durch Absorption der Substanz mittels eines flüssigen, meist wässrigen Trägers erfolgen, in dem die Substanz vorzugsweise gelöst ist. Bei der Einarbeitung der Wundheil- oder Hautpflegesubstanz im Zuge der Weiterverarbeitung ist es bevorzugt, dass die Substanz in einer flüssigen, vorzugsweise wässrigen Phase in das wasserabsorbierende Polymer, ggf. während der "Nachvernetzung" eingearbeitet wird.

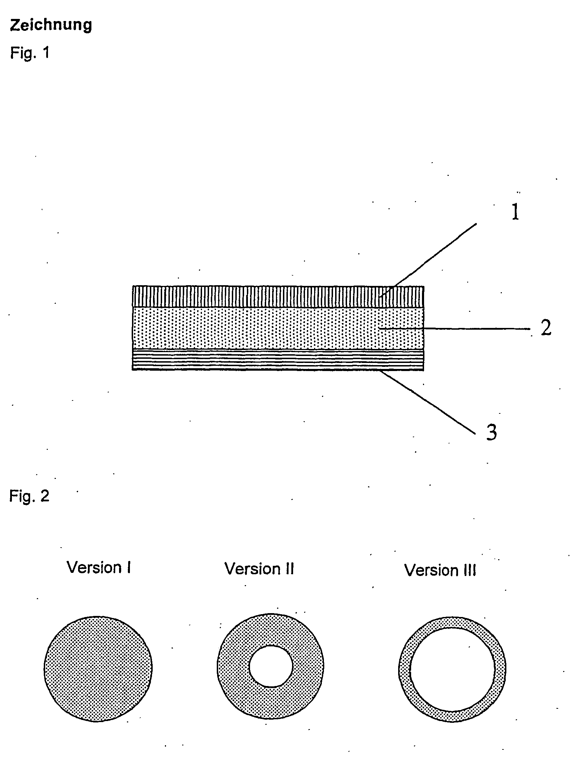

Es sind auch Kombinationen der vorstehenden Verfahrensvarianten möglich. Bei der Einarbeitung der Substanz vor Abschluss der Bildung des wasserabsorbierenden Polymers wird vorzugsweise eine gleichmäßige Dotierung des wasserabsorbierenden Polymers erreicht, d.h. vorteilhafterweise liegt die Substanz homogen verteilt im Polymer vor. Wird die Substanz nach Abschluss der Bildung des wasserabsorbierenden Polymers oder während dessen Weiterverarbeitung oder beides eingearbeitet, so wird vorzugsweise eine Dotierung des wasserabsorbierenden Polymerteilchens in dessen Außen- oder Oberflächenbereich erhalten, wie es Fig. 2 erläutert. Eine Kombination der beiden Verfahrensvarianten führt in aller Regel zu einem Polymer mit einer unterschiedlichen Konzentration im innen- und Außenbereich des wasserabsorbierenden Polymerteilchens, wobei die Konzentration an Wundheil- bzw. Hautpflegesubstariz im Außenbereich meist höher ist.

Das so erhaltene mit der Substanz dotierte wasserabsorbierende Polymer, nachfolgend "dotiertes oder inkorporiertes Polymer" genannt, kann anschließend in die Polymermatrix, bevorzugt in die Polyurethanmatrix, eingearbeitet werden. Es ist bevorzugt, dass das dotierte Polymer vor Abschluss der Bildung, d. h. bevor im wesentliche alle reaktiven funktionellen Gruppen eines Polyurethanmatrixmonomers abreagiert sind, der Polykondensatmatrix in diese eingearbeitet werden. Dieses erfolgt vorzugsweise dadurch, dass das dotierte Polymer dem zur Bildung der Polyurethantmatrix notwendigen Polyol beigegeben werden kann.
Der erfindungsgemäße Wundverband ermöglicht dem Fachmann damit erstmalig die Wirkstoffsubstanzen schon am Anfang der Polyurethanbildungsreaktion zuzuführen. Dieses bietet ihm großartige Vorteile im Bezug auf Variationsbreite und Menge der eingesetzten Wirkstoffe und erhöht die Flexibilität bei der Produktion der Wundverbände.

Ein Wundverband, der die gewünschten Eigenschaften aufweist, wird zum Beispiel erhalten indem eine Mischung folgender Einsatzstoffe durch einen geeigneten Katalysator vernetzt und flächig ausgestrichen wird:

| | |
|---|---|
| Polyetherpolyol | :334 g |
| Vernetzer mit 10 % Wundheilsubstanz (z.B. Dexpanthenol) | : 29 g |
| dotierter Superabsorber | : 37 g |
| Katalysator | : 0,8 g |

Wird der so erhältliche Wundverband auf eine Wunde aufgebracht, verursacht die Wundflüssigkeit eine Quellung des wasserabsorbierenden Polymers. Die Wundflüssigkeit wird von der Polyurethanmatrix und den darin enthaltenden wasserabsorbierenden Polymeren aufgenommen. Letztere beginnen in Kontakt mit der Flüssigkeit zu quellen. Durch diesen Quellvorgang wird der oder die wundheilungsfördernden Substanzen in den wasserabsorbierenden Polymeren freigesetzt. Damit wird eine direkt Freisetzung des Wirkstoffes an den Behandlungsort der Wunde ermöglicht. Dies ist ein überaus guter Anwendungsvorteil, der die Herstellung eines sog. "Intelligenten Pflasters" ermöglicht, das erst bei Kontakt mit Wundflüssigkeit den erforderlichen wundheilungsfördernden Wirkstoff freisetzt.

Die Kinetik der Freisetzung kann dabei einerseits über die Konzentration der wundheilungsfördernden Substanz in dem wasserabsorbierenden Polymer und andererseits über die Konzentration des wasserabsorbierenden Polymers in der Polyurethanmatrix gesteuert werden. Darüber hinaus wird die Freisetzung über die Verteilung der Substanz in dem wasserabsorbierenden Polymer beeinflusst (Fig. 2). Die Verteilung der Wirksubstanz im wasserabsorbierenden Polymer erfolgt erfindungsgemäß wie zuvor beschrieben in Abhängigkeit des Zugabezeitpunktes vor, nach oder während des Abschlusses der Bildung des wasserabsorbierenden Polymers. Bevorzugt ist ein Wundverband, wobei der Wirkstoff über das gesamte wasserabsorbierende Polymer, bevorzugt homogen, verteilt ist (Fig. 2 Version I).

Damit ist einzig über eine trockene Lagerung ein jederzeit einsatzbereiter Wundverband für den Verbraucher erhältlich, der neben den bekannten Vorteilen hydroaktiver Polyurethanwundverbände nur dort wundheilungsfördernde Wirkung entfaltet, wo diese erforderlich ist.

Des weiteren erlaubt das Herstellungsverfahren, das Inkorporieren der Wirkstoffe über die Verkapselung im Superabsorber, überhaupt erst die direkte Einarbeitung wundheilungsfördernder Substanzen in die Polyurethanmatrix vor deren Bildungsreaktion. Dadurch können überhaupt erst homogen mit Wirkstoffen dotierte Produktaufbauten realisiert werden, deren Formgebung während der Vernetzungsreaktion erfolgt.

Weiterhin bevorzugt ist, dass das wasserabsorbierende Polymer des Wundverbandes mindestens eine der nachfolgenden Eigenschaften aufweist:

A1) eine Partikelgrößenverteilung aufweisen, wobei mindestens 80 Gew.% der Partikel eine Partikelgröße in einem Bereich von 10 μm bis 900 μm nach ERT 420.1-99 besitzen;
A2) eine *Centrifuge Retention Capacity* (CRC) von mindestens 10 g/g, vorzugsweise mindestens 20 g/g nach ERT 441.1-99;
A3) eine *Absorption Against Pressure* (AAP) bei 0,7 psi von mindestens 4 g/g nach ERT 442-1.99;
A4) einen Gehalt an wasserlöslichem Polymer nach 16 Stunden Extraktion von weniger als 25 Gew.-%, jeweils bezogen auf das Gesamtgewicht des wasserabsorbierenden Polymers, nach ERT 470.1-99,
A5) eine Restfeuchtigkeit von höchstens 15 Gew.-%, jeweils bezogen auf das Gesamtgewicht des wasserabsorbierenden Polymers, nach ERT 430.1-99,

wobei die angegebenen Meßmethoden zur Partiketgrößenbestimmung bekannt sind.

Beispielhaft seien die folgenden Parameter zur Charakterisierung der Wundauflage bei Zusatz von 10% Superabsorber genannt:
Haftfähigkeit: rheologische Charakterisierung der visko-elastischen Eigenschaften

| | |
|---|---|
| tan δ (ω=0,3rad/s): Flüssigkeitsmanagement | 0,20 |
| Flüssigkeitsaufnahme: | 10g/100cm2 |
| Wasserdampfdurchlässigkeit: | 250 g / (m2 x 24h) |
| 02-Durchlässigkeit: | 2000 cm3 / (m2 x 24h) |

Die dargestellten Eigenschaften sind mit folgenden Testmethoden charakterisierbar.

- ERT
  Sofern nicht nachfolgend anderslautend dargestellt, werden ERT-Methoden zur Bestimmung der verschiedenen das wassserabsorbierende Polymer betreffenden Eigenschaften eingesetzt. ERT steht für EDANA Recommended Test, wobei EDANA für European Nonwoven and Diaper Association steht.
- <u>Extraktionstest</u>
  0,5g einer wirkstoffdotierten Probe, beispielsweise eine teilneutralisierte, schwach vernetzte Polyacrylsäure mit Dexpanthenol als Wirkstoff oder eine diese beinhaltende Polyurethanmatrix, wurden in eine 125ml Weithalsflasche auf einer Analysenwaage eingewogen. Nach Zugabe von 100ml 0,9%iger Kochsalzlösung (basierend auf destilliertem Wasser) und einem Tropfen konzentrierter Phosphorsäure wurde über eine Stunde auf dem Magnetrührer bei 350 Umdrehungen pro Minuten gerührt. Anschließend wurden 2ml der Lösung entnommen und durch einen 0,45μm Cellulosemischester-Membranfilter in ein Probenvial filtriert. Das Filtrat wurde dann einer HPLC-Analyse zugeführt,

wobei die in der HPLC-Analyse verwendete Probe einen sauren pH-Wert im Bereich von 2,5 bis 3,0 aufwies.
Der Gehalt an Wirkstoff wurde an Hand der HPLC-Analyseergebnisse durch externe Kalibrierung bestimmt. Hierzu wurde der zu bestimmende Wirkstoff mittels Analysenwaage in einem 100ml fassenden Messkolben mit mindestens 10mg auf 0,1mg genau eingewogen. Anschließend wurde der Messkolben mit ultrareinem Wasser bis zur Marke aufgefüllt. Entsprechend der Konzentration der entstandenen Stammlösung wird nun auf der Analysewaage eine Verdünnungsreihe hergestellt. Mittels dieser Verdünnungsreihe wird eine Kalibrierkurve durch HPLC-Analysen erstellt. Die Menge des über eine Stunde extrahierten Wirkstoffs wird durch den Vergleich der HPLC-Analyseergebnisse des entsprechenden Wirkstoffs mit der Kalibrierkurve ermittelt.
Die Chromatographischen Bedingungen wurden in Abhängigkeit des zu bestimmenden Wirkstoffs optimiert. Im Fall des Dexpanthenols wurde eine Säule des Typs GromSil 300 ODS-5 5μm (250 x 4mm) verwendet. Das Eluent wurde hergestellt, indem 13,61g $KH_2PO_4$ in ein 3l fassendes Becherglas eingewogen und nach Zugabe von 2000ml ultrareinem Wasser gelöst wurden. Anschließend wurde mit konzentrierter Phosphorsäure ein pH-Wert von 2,5 bis 3,0 eingestellt. Im Fall von Dexpanthenol wurde ein Fluss von 0,8ml/min. eingestellt. Die Injektion erfolgte über ein Loop von 20μl.

- Rheologische Charakterisierung
  Aus einem Pflaster wird mittig eine Probe mit einem Durchmesser von 8mm ausgestanzt und eine Stunde bei 23±2°C und 50±5%r.F. vorkonditioniert. Die Probe wird mittig auf einem 8mm Platte Drehkörper aufgeklebt und an einem schubspannungsgesteuerten Rheometermit mit einem Peltierelement zur Temperierung (z.B. RS-75 von HAAKE) vermessen. Dazu wird die Probe mit einer Normalkraft von 1,3N auf die untere Platte gedrückt. Nach einer Konditionierung von 5 Minuten bei 25±0,2°C werden bei einer Schubspannung von 700Pa die viskoelastischen Eigenschaften (Speicher- und Verlustmodul) im Frequenzbereich von $\varphi$= 0,3 bis 30 rad/s bestimmt.
  Der tanδ wird aus dem Quotienten aus Verlust-und Speichermodul berechnet.
- Flüssigkeitsaufnahme
  Aus einem Pflaster wird mittig eine Probe mit einem Durchmesser von 15 mm ausgestanzt und eine Stunde bei 23 ± 2 °C und 50 ± 5 % r.F. vorkonditioniert. Die Proben werden gewogen und für 3 Stunden vollständig in physiologische 23 ± 0,5 °C warme Natriumchloridlösung getaucht. Die Proben werden erneut gewogen und die Flüssigkeitsaufnahme aus der Wägedifferenz berechnet.
- Wasserdampfdurchlässigkeit
  Die Prüfung erfolgt nach ASTM E 96 (water method), mit folgenden Abweichungen:
  Die Öffnung des Prüfgefäßes beträgt 804 $mm^2$
  Das Material wird 24 Std. vorkonditioniert bei 23 ± 2 °C und 50 ± 5 % r.F.
  Der Abstand zwischen Wasserspiegel im Prüfgefäß und der Probe beträgt 35 ± 5 mm
  Das Rückwiegen der mit Proben bestückten Prüfgefäße erfolgt nach 24 Std., in denen diese im Klimaschrank bei 37 ± 1,5 °C und 30 ± 3 % r.F. gelagert werden.
- 02-Durchlässigkeit
  Prüfung gemäß ASTM D3985-8

Bevorzugt ist weiterhin, dass das wasserabsorbierende Polymer ein Partikelgrößenverteilung zwischen 10 und 500 μm und/oder eine Restfeuchte kleiner 10 Gew.%, bevorzugt kleiner gleich 3 Gew.%, besitzt.
Die vorstehenden Teilchengrößenverteilungen und Teilchengrößen sowie Restfeuchten sind insbesondere für eine gleichmäßige Abgabe und Verteilung der Wirkstoffe und für einen guten Tragekomfort vorteilhaft. Außerdem hat sich gezeigt, dass die vorstehenden Teilchengrößenverteilungen und Teilchengrößen sich besonders gut in flexible Matrices einarbeiten lassen, die, wenn in Pflaster oder Wundauflagen eingearbeitet, die Anpassungsfähigkeit dieser Pflaster oder Wundauflagen an die Form der Wunde und an deren Bewegungen erhöht.
Schließlich kann die Matrix mit einem klebstoffabweisenden Trägermaterial, wie silikonisiertem Papier, eingedeckt oder mit einer Wundauflage oder einer Polsterung versehen werden. Auf seiner, bevorzugt selbstklebend ausgerüsteten, später der Haut zugewandten Seite ist der erfindungsgemäße Verband über seine ganze Breite bis zum Gebrauch üblicherweise mit einem klebstoffabweisenden Trägermaterial abgedeckt. Dieses schützt die Selbstklebeschicht aus der gut hautverträglichen Klebemasse der Matrix, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.
Der erfindungsgemäße Wundverband, zumeist in Form eines Pflasters, umfasst bevorzugt eine erfindungsgemäße selbstklebende wirkstoffhaltige Polyurethanmatrix, eine wirkstoffundurchlässige Rückschicht und eine ablösbare Schutzschicht, die vor dem Applizieren auf der Haut entfernt wird. Weitere Ingredienzien, wie Füllstoffe, Stabilisatoren, Enhancer und/oder kosmetische Zusätze können in der Matrix eingearbeitet werden, um den Verband an die unterschiedlichen Anwendungsgebiete anzupassen und um einen anwendungsfreundlichen Verband bereit zu stellen.
Zur Erläuterung zeigt

Fig. 1 eine bevorzugte Ausführungsform des Wundverbandes
Fig. 2 die Verteilung des Wirkstoffen im wasserbasorbierenden Polymer in den Versionen I, II und III (s. Beispiele).

Aufgebaut ist ein entsprechendes Pflaster aus einem Träger wie Folien, Vliese, Gewebe, Schäume (1) etc., der Klebmatrix (2) und Abdeckfolie, Abdeckmaterial oder Trennpapier (3) zum Schutz der klebenden Matrix vor .dem Gebrauch des Pflasters, wie es in Abbildung 1 dargestellt ist.
In einer weiteren bevorzugten Ausführungsform der Erfindung werden als Träger Polymerfolien, Vliese, Gewebe sowie deren Kombinationen eingesetzt. Als Trägermaterialien stehen u.a. Polymere wie Polyethylen, Polypropylen, Polyester, Polyether-ester und Polyurethan oder auch Naturfasern zur Auswahl. Die Dicke der jeweiligen Schichten (1, 2, 3) liegen im Bereich von

(1) 10-150μm
(2) 50-2000μm
(3) 20-200μm

Zusammenfassend kann festgehalten werden, dass als Trägermaterialien sich alle starren und elastischen Flächengebilde aus synthetischen und natürlichen Rohstoffen eignen. Bevorzugt sind Trägermaterialien, die so eingesetzt werden können, dass sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona und Hydrophobieren; geläufige Nachbehandlungen sind Kalandern, Tempern, Kaschieren, Stanzen und Eindecken. Besonders vorteilhaft ist, wenn das Trägermaterial sterilisierbar, bevorzugt γ-(gamma) sterilisierbar, ist.
Eine durchgeführte γ-Sterilisation (Dosis=30kGy) zeigte keinen Einfluß auf den erfindungsgemäßen Wundverband.
Die genannten Eigenschaften der Klebmatrix legen insbesondere die Verwendung der erfindungsgemäßen Wundverbände für medizinische Produkte, insbesondere Pflaster, medizinische Fixierungen, Wundabdeckungen, orthopädische oder phlebologische Bandagen und Binden nahe.
Die erfindungsgemäßen Wundverbände sind in der Lage Wundexudat sowie Feuchtigkeit von der Haut aufzusaugen und gegebenenfalls durch das Pflaster nach außen abzutransportieren. Damit wird ein optimales feuchtes Wundheilungsmilieu erzeugt. Aufgrund der Hautverträglichkeit und der schmerzfreien Wiederablösbarkeit sind darüber hinaus für den Anwender wesentliche Vorrausetzungen für die Verwendung des erfindungsgemäßen Wundverbandes gegeben.
Neben der Anwendung als Verband, Pflaster oder Bandagenmaterial kann die erfindungsgemäße Auflage auch als Hautpflegeprodukt eingesetzt werden. Dazu können neben den erfindungsgemäß vorhanden mit Wirkstoff dotierten Superabsorber weitere, insbesondere die Haut pflegende, befeuchtende oder heilende Stoffe eingebaut werden. Darüber hinaus ist die Auflage als feuchtes oder trockenes Kosmetiktuch oder Pad zu verwenden.

Beispiele

A. Herstellung von Labormustern mit Dexpanthenol-dotiertem Superabsorber

1. Vorbereitung der zweier Massen

| | | |
|---|---|---|
| Komponente 1: | 82 Gew.% | Polyether-Polyol*<br>Levagel E (Bayer AG) |
| | 9 Gew.% | Isocyanat-Prepolymer**<br>Desmodur (Bayer AG) |
| | 9 Gew.% | Favor T, dotiert mit 10% Dexpanthenol<br>(Degussa Stockhausen AG) |

einwiegen und 24 Stunden auf dem Rollbock mischen

| | | |
|---|---|---|
| Komponente 2: | 90 Gew.-% | Polyether-Polyol* |
| | 10 Gew.-% | Katalysator*** |

(fortgesetzt)

| CosCat (CasChem Inc.) |
| --- |
| * Pentaerythrit + Propylenoxid + Ethylenoxid-Mischpolymerisat mit Ethylenoxid-Endblock Funktionalität: 4, OH-Zahl: 35, mittleres Molgewicht: 6400 (berechnet). Viskosität (23°C): 1000 mPas, Ethylenoxid-Gehalt: 20 Gew.-% ** NCO-terminiertesPrepolymer aus Umsatz bei 80 °C von Hexamethylendiisocyanat und Polypropylenglykol (mittleres Molekulargewicht: 220) im Molverhältnis 5:1 und ansschließender Vakuumdestillation bei ca. 0,5 mbar bis auf einen HDI-Monomer-Restgehalt < 0,5 Gew.-% NCO-GEhalt: 12,6 Gew.-%, Viskosität (23 °C): 5000 mPas *** Lösung von 1 mol des Bi(III)Salzes mit 2,2-Dimethyloctansäure in 3 mol 2,2-Dimethyloctansäure (Wismutgehalt ca. 17 Gew.-%) |

2. Herstellung von Ausstrichen

| | |
| --- | --- |
| Einsatzstoffe: | Komponente 1 |
| | Komponente 2 |

Folie mit einer Wasserdampfdurchlässigkeit von ca. 750 g/($m^{2*}$d)

| | | |
| --- | --- | --- |
| Durchführung: | 98 Gew.% | Komponente 1 |
| | 2 Gew.% | Komponente 2 |

einwiegen und ca. 40 s vermischen, auf Trennpapier gießen, vor dem Streichbalken die Folie zukaschieren, PU-Masse mit dem Streichbalken zwischen Trennpapier und Folie ausstreichen:
Spalteinstellung am Streichbalken 1 mm
Aushärtung der verstrichenen PU-Masse für 5 min bei 65°C
Aus dem Ausstrich (Flächengewicht ca. 850 g/$m^2$) können Auflagen ausgestanzt werden

B. Nachfolgend wird beispielhaft die Herstellung eines erfindungsgemäßen Wundverbandes erläutert.

A. Herstellung mit Dexpanthenol dotierter Superabsorber auf Polyacrylsäurebasis

Die Herstellung des Superabsorbers verläuft nach gängigen Methoden indem bei einer Temperatur von ca. 150°C die Polymerisation wässriger Acrylsäurelösung gestartet wird.
Der Wassergehalt der Lösung beträgt ca. 70 Gew.%. An dieser Stelle (I) ist der Zusatz des Dexpanthenols in die Polymerisationlösung bereits möglich. Es entsteht ein mit Dexpanthenol dotierter Superabsorber der Version (I) wie in Fig.2 dargestellt. Das entstandene Polymerisat wird zerkleinert und bei ca. 150°C getrocknet. Anschließend kann ebenfalls Dexpanthenol zugegeben werden (II), so dass ein mit Dexpanthenol dotierter Superabsorber der Version (II) entsteht. Diese verspätete Zugabe an Dexpanthenol hat den Vorteil, dass das Dexpanthenol der vorherigen Trocknung nicht ausgesetzt worden ist. Das Polymerisat wird weiter gemahlen und ggf. oberflächenmodifiziert und getrocknet. An dieser Stelle (III) kann ebenfalls das Dexpanthenol zugesetzt werden. Es entsteht ein mit Dexpanthenol dotierter Superabsorber der Version (III). Anschließend wird das Polymerisat bei ca. 150°C bis zu einer Restfeuchte von ca. 7% bis 10% getrocknet.
Vorteilhafterweise schließen sich nun zwei weitere Verfahrensschritte (IV und V) an, die einerseits die Partikelgröße und andererseits die Restfeuchte des mit Dexpanthenol dotierten Superabsorbers optimieren.
Danach wird das nach dem Verfahrensschritt (III) getrocknete und mit Dexpanthenol dotierte Polymerisat gemahlen. Der Superabsorber (IV) weist dann eine Partikelgrößenverteilung von ca. 10 bis 500 $\mu$m, bevorzugt 20 bis 200 $\mu$m, auf. Als letzter Verfahrensschritt (V) wird der dotierte Superabsorber erneut getrocknet. Die Trocknung führt zu einer Restfeuchte kleiner 10%, bevorzugt ≤ 3%, des dotierten Superabsorbers (V).

Im Vergleich der herstellbaren dotierten Superabsorber der Versionen I bis V werden einige Unterschiede deutlich, die in Abbildung 2 gezeigt sind. Bei gleicher Einsatzmenge an Dexpanthenol liegt das Dexpanthenol bei der Version (1) homogen verteilt in den Superabsorberpartikeln vor. Bei der Version (II) befindet sich das Dexpanthenol verteilt in einem äußeren Ring der Partikel und bei der Version (III) ist das gesamte Dexpanthenol nur auf der äußersten Schicht der Partikeloberfläche zu finden. Die beiden letzteren Versionen (II und III) führen dazu, dass aufgrund der Anhäufung des Dexpanthenols an der Oberfläche der Superabsorber zum Teil klebriger wird und daher die Verarbeitung ggf. schwieriger wird.

Die Version (IV), mit einer max. Partikelgröße von 500 μm und einer minimalen Partikelgröße oberhalb der Lungengängigkeit, stellt die optimale Partikelgrößenverteilung dar. Damit ist eine gute Weiterverarbeitung der dotierten Superabsorberpartikel gewährleistet.

Die Version (V) mit einer Restfeuchte kleiner 10% ist eine weitere optimierte Version der mit Dexpanthenol dotierten Superabsorber.

Als besonders bevorzugt für den Einsatz in den erfindungsgemäßen Wundverbänden hat sich demnach ein mit Dexpanthenol dotierter Superabsorber der Kombination der Versionen I, IV und V erwiesen. Bei diesem optimalen dotierten Superabsorber liegt das Dexpanthenol homogen verteilt in den Absorberpartikeln vor. Die Absorberpartikel haben ein Partikelgrößenverteilung zwischen 10 und 500 μm und besitzen eine Restfeuchte kleiner 10%, bevorzugt kleiner gleich 3%.

Durch die verschiedenen Herstellungsmöglichkeiten (Version I, II und/oder III gemäß Fig. 2) schafft man eine Variationsbreite auch in der Freisetzungskinetik des Wirkstoffes. Aus Version 1 wird eine langandauernde Freisetzung generiert, wobei vorteilhafterweise eine homogene Verteilung des Wirkstoffes im Polymer vorliegt. Durch Kombination der einzelnen Herstellungsschritte können somit Freisetzungsbereiche der Wirkstoffe gezielt eingestellt werden. So kann der Wirkstoff in relativ kurzer Zeit und hoher Dosis bis zu langandauernder Abgabe und geringer Dosis freigegeben werden.

B. Herstellung des Wundverbandes

Diese dotierten Superabsorberpartikel werden nun direkt in die Ausgangsmischung der Polyurethanreaktion zugesetzt. Sowohl die gleichmäßige Verteilung des Dexpanthenols im Superabsorber (Version 1) als auch eine Restfeuchte kleiner gleich 3% (Version V) führt zu keiner den Herstellprozeß störenden Beeinträchtigung der Polyurethanbildung. Die Polyurethanbildung verläuft damit störungsfrei, wie oben beschrieben. Die den dotierten Superabsorber enthaltene Polyurethanmatrix wird anschließend auf einem Trennpapier ausgegossen und mit einer Polyurethanfolie abgedeckt.

Diese zwischen Polyurethanfolie und Trennpapier eingedeckte Polyurethanmatrix wird als Ballen gefertigt und auf Mutterrollen in die entsprechende Bahnenbreite gebracht. Aus den Bahnen können die Wundauflagen entsprechender Größe ausgestanzt werden. Diese selbstklebenden Wundauflagen können selbst als Pflaster verwendet werden und haben demnach einen der Abbildung 1 entsprechenden Aufbau.

In einem weiteren Verfahrensschritt können diese Polyurethanwundauflagen mit dotierten Superabsorbern auf einem Trägermaterial mit einer Klebemasse aufgelegt werden. Aus diesem Träger kann abschließend das fertige Pflaster mit Kleberand entsprechender Größe ausgestanzt werden. Als Trägermaterialien kommen die in der Pflastertechnologie bekannten Materialien, wie Folien aus Polyurethan, Polyethylen, Polypropylen, Polyamid, Polyester oder Polyetherester sowie Gewebe, Vlies, nonwoven, Gewirke, Gelege, Laminate, Netze, Folien, Schäume oder Papiere in Betracht. Als Klebemasse können ebenfalls alle bekannten Klebemassen, wie Acrylat-, hot-melt-Polyurethan verwendet werden.

Die bei der Herstellung verwendete Polyurethanfolie ist optional. Ebenso läßt sich die Polyurethanmatrix nur auf dem Trennpapier auftragen und weiterverarbeiten, was dazu führt, dass die Polyurethanfolie dann im fertigen Wundverband nicht vorhanden ist.

## Patentansprüche

**1.** Haut- oder Wundauflage beinhaltend

a. eine Polykondensatmatrix basierend auf mindestens einem Polykondensatmonomer mit mindestens einer Polykondensatgruppe, und
b. ein teilchenförmiges wasserabsorbierendes Polymer, beinhaltend mindestens eine wundheilungsfördemde und/oder mindestens eine hautpflegende Substanz, mit mindestens einer funktionellen Gruppe, die mit der Polykondensatgruppe unter Bildung einer kovalenten Bindung reagieren könnte, oder
c. ein wasserabsorbierendes Polymer enthaltend mindestens eine wundheilungsfördemde und/oder mindestens

eine hautpflegende Substanz,

wobei das teilchenförmige wasserabsorbierende Polymer mindestens teilweise von der Polykondensatmatrix umgeben ist, und
wobei die Haut- oder Wundauflage eine Wundheilsubstanz- oder Wirkstoffverfügbarkeit von mindestens 10 Gew. % nach dem hierin angegebenen Extraktions-Test zeigt,
**dadurch gekennzeichnet, dass**
die wundheilungsfördernde und/oder hautpflegende Substanz in dem wasserabsorbierenden Polymer inkorporiert ist.

**2.** Haut- oder Wundauflage umfassend

a. eine luft- und wasserdampfdurchlässige Polyurethanmatrix enthaltend
b. ein wasserabsorbierendes Polymer in das mindestens eine wundheilungsfördernde und/oder mindestens eine hautpflegende Substanz inkorporiert ist.

**3.** Haut- oder Wundauflage nach Anspruch 2, **dadurch gekennzeichnet, dass** die Polyurethanmatrix gebildet wird aus

a) 2 bis 6 Hydroxylgruppen aufweisenden Polyetherpolyolen mit OH-Zahlen von 20 bis 112 und einem Ethylenoxid (EO)-Gehalt von ≥ 10 Gew.-%,
b) Antioxidantien,
c) in den Polyolen a) löslichen Wismut-(III)-Carboxylaten auf Basis von Carbonsäuren mit 2 bis 18 C-Atomen als Katalysatoren sowie
d) Hexamethylendiisocyanat,
e) mit einem Produkt der Funktionalitäten der Polyurethan bildenden Komponenten a) und d) von mindestens 5,2, wobei die Katalysatormenge c) 0,005 bis 0,25 Gew.-%, bezogen auf das Polyol a) beträgt, die Menge an Antioxidantien b) im Bereich von 0,1 bis 1,0 Gew,%, bezogen auf Polyol a) liegt und ein Verhältnis von freien NCO-Gruppen der Komponente d) zu den freien OH-Gruppen der Komponente a) im Bereich von 0,30 bis 0,70 gewählt wird.

**4.** Haut- oder Wundauflage nach einem der vorstehenden Ansprüche 2 oder 3, **dadurch gekennzeichnet, dass** die Polyurethanmatrix selbstklebend ausgerüstet ist.

**5.** Haut- oder Wundauflage nach einem der vorstehenden Ansprüche, **dadurch gekennzeichnet, dass** eine oder mehrere wundheilungsfördemde Substanzen aus der Gruppe Dexpanthenol, Ringelblume, Hamamelis und/oder Kamille, bevorzugt Dexpanthenol, und eine oder mehrere hautpflegende Substanzen aus der Gruppe der Vitamine, Antioxidantien, Lichtschutzmittel, Insektenrepellentien, ätherische Öle, antimikrobielle Mittel, Moisturizer, Parfume und insbesondere Coenzym Q10 gewählt werden.

**6.** Haut- oder Wundauflage nach einem der vorstehenden Ansprüche, **dadurch gekennzeichnet, dass** die wundheilungsfördemde und/oder hautpflegende Substanzen zu 0,1 bis 10,0 Gew.%, bevorzugt 0,2 bis 5 Gew.%, bezogen auf die Matrix, enthalten sind.

**7.** Haut- oder Wundauflage nach einem der vorstehenden Ansprüche, **dadurch gekennzeichnet, dass** die wundheilungsfördernde und/oder hautpflegende Substanzen zu 0,001 bis 30 Gew.%. bevorzugt 5 bis 15 Gew.%, bezogen auf das wasserabsorbierende Polymer enthalten sind.

**8.** Haut- oder Wundauflage nach einem der vorstehenden Ansprüche, **dadurch gekennzeichnet, dass** das wasserabsorbierende Polymer vernetztes Natriumpolyacrylat enthält.

**9.** Haut- oder Wundauflage nach einem der vorstehenden Ansprüche, **dadurch gekennzeichnet, dass** das wasserabsorbierende Polymer zu mindestens 50 Gew.-%, vorzugsweise zu mindestens 70 Gew.-%, besonders bevorzugt zu mindestens 90 Gew.-% aus carboxylatgruppenhaltigen Monomeren besteht.

**10.** Haut- oder Wundauflage nach einem der vorstehenden Ansprüche, **dadurch gekennzeichnet, dass** das wasserabsorbierende Polymer zu mindestens 50 Gew.-%, vorzugsweise zu mindestens 70 Gew.-% aus Acrylsäure besteht, die vorzugsweise zu mindestens 20 Mol-%, besonders bevorzugt zu mindestens 50 Mol-%, besonders bevorzugt im Bereich von 65 bis 85 Mol-%, vorzugsweise mit Natronlauge, neutralisiert ist.

**11.** Haut- oder Wundauflage nach einem der vorstehenden Ansprüche, **dadurch gekennzeichnet, dass** das Polymer eine Absorbermatrix in einer Menge im Bereich von 70 bis 99,99 Gew.% bezogen auf das wirkstoffdotierte wasserabsorbierende Polymer, enthält,
wobei die Absorbermatrix zu mindestens 90 Gew.%, bezogen auf die Absorbermatix, aus einer vernetzten Polyacrylsäure besteht,
wobei die vernetzte Polyacrylsäure zu mindestens 90 Gew.%, bezogen auf die vernetzte Polyacrylsäure, aus mindestens 30 Mol.% teilneutralisierten Acrylsäure besteht.

**12.** Haut- oder Wundauflage nach einem der vorstehenden Ansprüche, **dadurch gekennzeichnet, dass** die Matrix, insbesondere Polyurethanmatrix, transparent eingestellt ist.

**13.** Haut- oder Wundauflage nach einem der vorstehenden Ansprüche, **dadurch gekennzeichnet, dass** die Matrix vollflächig oder partiell, geschäumt oder ungeschäumt auf einer Trägerfolie aufgetragen ist.

**14.** Haut- oder Wundauflage nach Anspruch 13, **dadurch gekennzeichnet, dass** die Trägerfolie aus Polyurethan, Polyethylen, Polypropylen, Polyamid, Polyester oder Polyether-ester besteht.

**15.** Haut- oder Wundauflage nach einem der vorstehenden Ansprüche, **dadurch gekennzeichnet, dass** die Matrix mit einer Abdeckfolie, Abdeckpapier oder Trennpapier zum Schutz der gegebenenfalls klebenden Matrix abgedeckt ist.

**16.** Haut- oder Wundauflage nach einem der vorstehenden Ansprüche, **dadurch gekennzeichnet, dass** der Wirkstoff über das gesamte wasserabsorbierende Polymer, bevorzugt homogen, verteilt ist.

**17.** Haut- oder Wundauflage nach einem der vorstehehenden Ansprüche, wobei das wasserabsorbierende Polymer mindestens eine der nachfolgenden Eigenschaften aufweist:

A1) eine Partikelgrößenverteilung aufweisen, wobei mindestens 80 Gew.-% der Partikel eine Partikelgröße in einem Bereich von 10 $\mu$m bis 900 $\mu$m nach ERT 420.1-99 besitzen:
A2) eine *Centrifuge Retention Capacity* (CRC) von mindestens 10 g/g, vorzugsweise mindestens 20 g/g nach ERT 441.1-99;
A3) eine *Absorption Against Pressure* (AAP) bei 0,7 psi von mindestens 4 g/g nach ERT 442-1.99;
A4) einen Gehalt an wasserlöslichem Polymer nach 16 Stunden Extraktion von weniger als 25 Gew.-%, jeweils bezogen auf das Gesamtgewicht des wasserabsorbierenden Polymers, nach ERT 470.1-99,
A5) eine Restfeuchtigkeit von höchstens 15 Gew.-%, jeweils bezogen auf das Gesamtgewicht des wasserabsorbierenden Polymers, nach ERT 430.1-99.

**18.** Haut- oder Wundauflage nach einem der vorstehenden Ansprüche, **dadurch gekennzeichnet, dass** das wasserabsorbierende Polymer ein Partikelgrößenverteilung zwischen 10 und 500 $\mu$m und/oder eine Restfeuchte kleiner 10 Gew.%, bevorzugt kleiner gleich 3 Gew.%, besitzt.

**19.** Verfahren zur Herstellung von Haut- oder Wundauflagen umfassend eine Polyurethanmatrix und ein wasserabsorbierendes Polymer in das mindestens eine wundheilungsfördemde und/oder hautpflegende Substanz inkorporiert ist, indem
eine Mischung eines Polyetherpolyols und eines Vernetzers auf Basis eines aliphatischen Isocyanatpräpolymers vernetzt wird und vor der Vernetzung ein mit einer wundheilungsfördernden und/oder hautpflegenden Substanz dotiertes wasserabsorbierendes Polymer zugegeben wird.

## Claims

**1.** Skin or wound contact material comprising

a. a polycondensate matrix based on at least one polycondensate monomer having at least one polycondensate group, and
b. a particulate, water-absorbing polymer comprising at least one wound healing promoter substance and/or at least one skincare substance having at least one functional group which could react with the polycondensate group and, in so doing, forms a covalent bond, or
c. a water-absorbing polymer comprising at least one wound healing promoter substance and/or at least one

skincare substance,

the particulate, water-absorbing polymer being at least partly surrounded by the polycondensate matrix, and the skin or wound contact material exhibiting a wound-healing substance or active substance availability of at least 10% by weight by the extraction test indicated herein,
**characterized in that**
the wound healing promoter substance and/or skincare substance is incorporated in the water-absorbing polymer.

**2.** Skin or wound contact material comprising

a. an air and water vapour permeable polyurethane matrix comprising
b. a water-absorbing polymer into which at least one wound healing promoter substance and/or at least one skincare substance is incorporated.

**3.** Skin or wound contact material according to Claim 2, **characterized in that** the polyurethane matrix is formed from

a) polyether polyols having 2 to 6 hydroxyl groups, OH numbers of 20 to 112, and an ethylene oxide (EO) content of ≥ 10% by weight,
b) antioxidants,
c) bismuth(III) carboxylates soluble in the polyols (a) and based on carboxylic acids having 2 to 18 carbon atoms, as catalysts, and
d) hexamethylene diisocyanate,
e) with a product of the functionalities of the polyurethane-forming components a) and d) of at least 5.2, the amount of catalyst c) being 0.005% to 0.25% by weight, based on the polyol a), the amount of antioxidants b) being in the range from 0.1% to 1.0% by weight, based on polyol a), and the selected ratio of free NCO groups of component d) to the free OH- groups of component a) being in the range from 0.30 to 0.70.

**4.** Skin or wound contact material according to one of Claims 2 or 3 above, **characterized in that** the polyurethane matrix is self-adhesive.

**5.** Skin or wound contact material according to any one of the above claims, **characterized in that** one or more wounds healing promoter substances from the group dexpanthenol, marigold, witch hazel and/or camomile, preferably dexpanthenol, and one or more skincare substances from the group of vitamins, antioxidants, light stabilizers, insect repellents, essential oils, antimicrobial agents, moisturizers, perfumes and, in particular, coenzyme Q10 are selected.

**6.** Skin or wound contact material according to any one of the above claims, **characterized in that** the wound healing promoter substances and/or skincare substances are present at 0.1% to 10.0% by weight, preferably 0.2% to 5% by weight, based on the matrix.

**7.** Skin or wound contact material according to any one of the above claims, **characterized in that** the wound healing promoter substances and/or skincare substances are present at 0.001% to 30% by weight, preferably 5% to 15% by weight, based on the water-absorbing polymer.

**8.** Skin or wound contact material according to any one of the above claims, **characterized in that** the water-absorbing polymer comprises crosslinked sodium polyacrylate.

**9.** Skin or wound contact material according to any one of the above claims, **characterized in that** the water-absorbing polymer is composed of at least 50%, preferably at least 70%, more preferably at least 90%, by weight of monomers containing carboxylate groups.

**10.** Skin or wound contact material according to any one of the above claims, **characterized in that** the water-absorbing polymer is composed of at least 50% by weight, preferably at least 70% by weight, of acrylic acid which is neutralized to an extent of preferably at least 20 mol%, more preferably at least 50 mol%, very preferably in the range from 65 to 85 mol%, preferably with aqueous sodium hydroxide solution.

**11.** Skin or wound contact material according to any one of the above claims, **characterized in that** the polymer comprises an absorber matrix in an amount in the range from 70% to 99.99% by weight, based on the active-doped water-absorbing polymer,

the absorber matrix being composed of at least 90% by weight, based on the absorber matrix, of a crosslinked polyacrylic acid,
the crosslinked polyacrylic acid being composed of at least 90% by weight, based on the crosslinked polyacrylic acid, of acrylic acid partly neutralized to at least 30 mol%.

**12.** Skin or wound contact material according to any one of the above claims, **characterized in that** the matrix, particularly polyurethane matrix, is transparent.

**13.** Skin or wound contact material according to any one of the above claims, **characterized in that** the matrix is applied in foamed or unfoamed form, partially or over the whole area, to a backing sheet.

**14.** Skin or wound contact material according to Claim 13, **characterized in that** the backing sheet is composed of polyurethane, polyethylene, polypropylene, polyamide, polyester or polyether-ester.

**15.** Skin or wound contact material according to any one of the above claims, **characterized in that** the matrix is lined with a liner sheet, liner paper or release paper for protecting the possibly adhesive matrix.

**16.** Skin or wound contact material according to any one of the above claims, **characterized in that** the active substance is distributed, preferably homogeneously, over the entire water-absorbing polymer.

**17.** Skin or wound contact material according to any one of the above claims, the water-absorbing polymer having at least one of the following properties:

A1) a particle size distribution with at least 80% by weight of the particles possessing a size in a range from 10 μm to 900 μm by ERT420.1-99;
A2) a *centrifuge retention* capacity (CRC) of at least 10 g/g, preferably at least 20 g/g, by ERT441.1-99;
A3) an *absorption against* pressure (AAP) at 0.7 psi of at least 4 g/g by ERT442.1-99;
A4) a water-soluble polymer content after 16 hours' extraction of less than 25% by weight, based in each case on the total weight of the water-absorbing polymer, by ERT470.1-99;
A5) a residual moisture content of not more than 15% by weight, based in each case on the total weight of the water-absorbing polymer, by ERT430.1-99.

**18.** Skin or wound contact material according to any one of the above claims, **characterized in that** the water-absorbing polymer possesses a particle size distribution of between 10 and 500 μm and/or a residual moisture content of less than 10% by weight, preferably less than or equal to 3% by weight.

**19.** Method of producing skin or wound contact materials comprising a polyurethane matrix and a water-absorbing polymer into which at least one wound healing promoter substance and/or skincare substance is incorporated, by crosslinking a mixture of a polyether polyol and a crosslinker based on an aliphatic isocyanate prepolymer and adding a water-absorbing polymer doped with a wound healing promoter substance and/or skincare substance prior to the crosslinking.

**Revendications**

**1.** Pansement applicable sur la peau ou les plaies, comprenant

a) une matrice d'un produit de polycondensation basé sur au moins un monomère d'un produit de polycondensation avec au moins un groupe d'un produit de polycondensation, et
b) un polymère absorbant l'eau sous forme de particules, comprenant au moins une substance favorisant la guérison de plaies et/ou au moins une substance de soin de la peau, présentant au moins un groupe fonctionnel qui est en mesure de réagir avec le groupe d'un produit de polycondensation avec formation d'une liaison covalente, ou
c) un polymère absorbant l'eau, contenant au moins une substance favorisant la guérison de plaies et/ou au moins une substance de soin de la peau,

le polymère absorbant l'eau en forme de particules étant au moins partiellement entouré de la matrice de produit de polycondensation, et

le pansement applicable sur la peau ou sur les plaies présentant une disponibilité de substance de guérison de plaies ou de substance active d'au moins 10% en poids selon le test d'extraction indiqué ici, **caractérisé en ce que** la substance favorisant la guérison de plaies et/ou de soin de la peau est incorporée dans le polymère absorbant l'eau.

**2.** Pansement applicable sur la peau ou sur les plaies, comprenant

a) une matrice de polyuréthane perméable à l'air et à la vapeur d'eau, contenant
b) un polymère absorbant l'eau dans lequel est incorporé au moins une substance favorisant la guérison des plaies et/ou au moins une substance de soin de la peau.

**3.** Pansement applicable sur la peau ou les plaies selon la revendication 2, **caractérisé en ce que** la matrice de polyuréthane est formée à partir

a) de polyétherpolyols présentant 2 à 6 groupes hydroxyle avec des indices OH de 20 à 112 et une teneur en oxyde d'éthylène (OE) $\geq$ 10% en poids,
b) d'antioxydants,
c) de carboxylates de bismuth (III) solubles dans les polyols a) à base d'acides carboxyliques comprenant 2 à 18 atomes de carbone comme catalyseur ainsi que
d) d'hexaméthylènediisocyanate,
e) présentant un produit des fonctionnalités des composants formant le polyuréthane a) et d) d'au moins 5,2, la quantité de catalyseur c) étant de 0,005 à 0,25% en poids, par rapport au polyol a), la quantité d'antioxydants b) se situant dans la plage de 0,1 à 1,0% en poids, par rapport au polyol a) et en choisissant un rapport de groupes NCO libres du composant d) aux groupes OH libres du composant a) dans la plage de 0,30 à 0,70.

**4.** Pansement applicable sur la peau ou les plaies selon l'une quelconque des revendications précédentes 2 ou 3, **caractérisé en ce que** la matrice de polyuréthane est apprêtée de manière auto-adhésive.

**5.** Pansement applicable sur la peau ou les plaies selon l'une quelconque des revendications précédentes, **caractérisé en ce qu'**on choisit une ou plusieurs substances favorisant la guérison de plaies dans le groupe formé par le Dexpanthenol, le souci officinal, l'hamamélis et/ou la camomille, de préférence le Dexpanthenol, et une ou plusieurs substances de soin de la peau dans le groupe formé par les vitamines, les antioxydants, les agents de protection contre la lumière, les agents répulsifs pour les insectes, les huiles essentielles, les agents antimicrobiens, les agents d'hydratation, les parfums et en particulier la coenzyme Q10.

**6.** Pansement applicable sur la peau ou les plaies selon l'une quelconque des revendications précédentes, **caractérisé en ce que** les substances favorisant la guérison de plaies et/ou de soin de la peau sont contenues à raison de 0,1 à 10,0% en poids, de préférence de 0,2 à 5% en poids, par rapport à la matrice.

**7.** Pansement applicable sur la peau ou les plaies selon l'une quelconque des revendications précédentes, **caractérisé en ce que** les substances favorisant la guérison de plaies et/ou de soin de la peau sont contenues à raison de 0,001 à 30% en poids, de préférence de 5 à 15% en poids, par rapport au polymère absorbant l'eau.

**8.** Pansement applicable sur la peau ou les plaies selon l'une quelconque des revendications précédentes, **caractérisé en ce que** le polymère absorbant de l'eau contient du polyacrylate de sodium réticulé.

**9.** Pansement applicable sur la peau ou les plaies selon l'une quelconque des revendications précédentes, **caractérisé en ce que** le polymère absorbant l'eau est constitué par au moins 50% en poids, de préférence par au moins 70% en poids, de manière particulièrement préférée par au moins 90% en poids de monomères contenant des groupes carboxylate.

**10.** Pansement applicable sur la peau ou les plaies selon l'une quelconque des revendications précédentes, **caractérisé en ce que** le polymère absorbant l'eau est constitué par au moins 50% en poids, de préférence par au moins 70% en poids d'acide acrylique, qui est de préférence neutralisé à raison d'au moins 20% en mole, de manière particulièrement préférée d'au moins 50% en mole, de manière particulièrement préférée dans la plage de 65 à 85% en mole, de préférence avec de la lessive de soude caustique.

**11.** Pansement applicable sur la peau ou les plaies selon l'une quelconque des revendications précédentes, **caractérisé en ce que** le polymère contient une matrice d'absorbant en une quantité dans la plage de 70 à 99,99% en poids

par rapport au polymère dopé de substance active, absorbant l'eau,
la matrice d'absorbant étant constituée par au moins 90% en poids, par rapport à la matrice d'absorbant, d'un poly (acide acrylique) réticulé,
le poly(acide acrylique) réticulé étant constitué par au moins 90% en poids, par rapport au poly(acide acrylique) d'acide acrylique partiellement neutralisé à au moins 30% en mole.

**12.** Pansement applicable sur la peau ou les plaies selon l'une quelconque des revendications précédentes, **caractérisé en ce que** la matrice, en particulier la matrice de polyuréthane est réglée de manière à être transparente.

**13.** Pansement applicable sur la peau ou les plaies selon l'une quelconque des revendications précédentes, **caractérisé en ce que** la matrice est appliquée sur toute la surface ou une partie de la surface, sous forme moussée ou non, sur une feuille support.

**14.** Pansement applicable sur la peau ou les plaies selon la revendication 13, **caractérisé en ce que** la feuille support est constituée par du polyuréthane, du polyéthylène, du polypropylène, du polyamide, du polyester ou du polyétherester.

**15.** Pansement applicable sur la peau ou les plaies selon l'une quelconque des revendications précédentes, **caractérisé en ce que** la matrice est recouverte par une feuille de protection, un papier de protection ou un papier de séparation pour la protection de la matrice le cas échéant adhésive.

**16.** Pansement applicable sur la peau ou les plaies selon l'une quelconque des revendications précédentes, **caractérisé en ce que** la substance active est répartie sur tout le polymère absorbant l'eau, de préférence de manière homogène.

**17.** Pansement applicable sur la peau ou les plaies selon l'une quelconque des revendications précédentes, le polymère absorbant l'eau présentant au moins l'une des propriétés suivantes :

A1) une répartition des grosseurs des particules, au moins 80% en poids des particules présentant une grosseur dans la plage de 10 µm à 900 µm selon la norme ERT 420.1-99 ;
A2) une capacité de rétention sous l'effet d'une force centrifuge (Centrifuge Retention Capacity - CRC) d'au moins 10 g/g, de préférence d'au moins 20 g/g selon la norme ERT 441.1-99 ;
A3) une absorption sous l'effet d'une pression (Absorption Against Pressure - AAP) à 0,7 psi d'au moins 4 g/g selon la norme ERT 442-1.99 ;
A4) une teneur en polymère soluble dans l'eau après 16 heures d'extraction inférieure à 25% en poids, à chaque fois par rapport au poids total du polymère absorbant l'eau, selon la norme ERT 470.1-99;
A5) une humidité résiduelle d'au maximum 15% en poids, à chaque fois par rapport au poids total du polymère absorbant l'eau, selon la norme ERT 430.1-99.

**18.** Pansement applicable sur la peau ou les plaies selon l'une quelconque des revendications précédentes, **caractérisé en ce que** le polymère absorbant l'eau présente une répartition des grosseurs des particules entre 10 et 500 µm et/ou une humidité résiduelle inférieure à 10% en poids, de préférence inférieure à 3% en poids.

**19.** Procédé pour la réalisation de pansements applicables sur la peau ou les plaies, comprenant une matrice de polyuréthane et un polymère absorbant l'eau dans lequel est incorporée au moins une substance favorisant la guérison de plaies et/ou au moins une substance de soin de la peau,
en ce que
on réticule un mélange d'un polyétherpolyol et d'un réticulant à base d'un prépolymère de type isocyanate aliphatique et on ajoute, avant la réticulation, un polymère absorbant l'eau, dopé avec une substance favorisant la guérison de plaies et/ou une substance de soin de la peau.

**Zeichnung**

Fig. 1

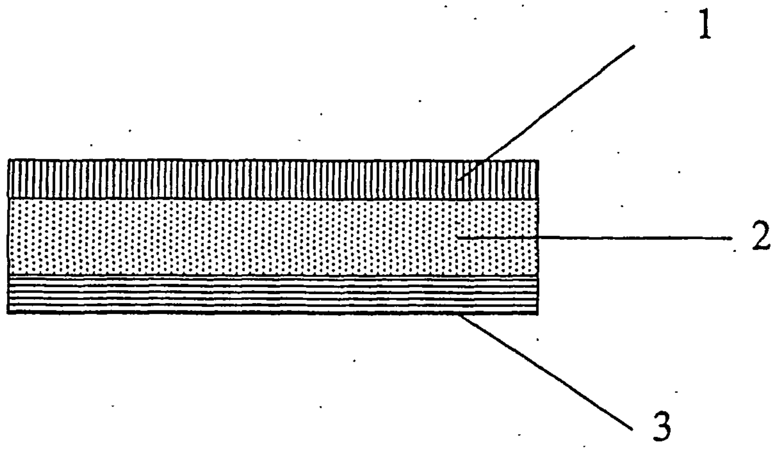


Fig. 2